(19) Europäisches Patentamt
European Patent Office
Office européen des brevets

(11) Publication number: **0 194 764**
**B1**

(12) **EUROPEAN PATENT SPECIFICATION**

(45) Date of publication of patent specification: **12.07.89**

(21) Application number: **86301074.0**

(22) Date of filing: **17.02.86**

(51) Int. Cl.$^4$: **C 07 C 103/30,**
C 07 D 307/79,
C 07 D 333/24,
C 07 D 333/60,
C 07 D 317/32,
C 07 D 311/74,
C 07 C 121/43, A 01 N 37/18,
A 01 N 45/00, A 01 N 43/12,
A 01 N 43/10

(54) **Pesticidal compounds.**

(30) Priority: **18.02.85 GB 8504097**
**25.02.85 GB 8504825**

(43) Date of publication of application:
**17.09.86 Bulletin 86/38**

(45) Publication of the grant of the patent:
**12.07.89 Bulletin 89/28**

(84) Designated Contracting States:
**AT BE CH DE FR GB IT LI LU NL SE**

(56) References cited:
**EP-A-0 111 105**
**EP-A-0 143 593**

**JOURNAL OF THE CHEMICAL SOCIETY, PERKIN TRANSACTION 1, 1982, pages 1493-1498, London, GB; B.G. PRING: "Isolation and identification of amides from Piper callosum. Synthesis of Pipercallosine and Pipercallosidine"**

**The file contains technical information submitted after the application was filed and not included in this specification**

(73) Proprietor: **THE WELLCOME FOUNDATION LIMITED**
**183-193 Euston Road**
**London NW1 2BP (GB)**

(72) Inventor: **Blade, Robert John**
**The Wellcome Research Laboratories**
**Berkhamsted Hertfordshire (GB)**
Inventor: **Black, Malcolm Henry**
**The Wellcome Research Laboratories**
**Berkhamsted Hertfordshire (GB)**
Inventor: **Weston, John Bernard**
**The Wellcome Research Laboratories**
**Berkhamsted Hertfordshire (GB)**
Inventor: **Larkin, Patrick**
**The Wellcome Research Laboratories**
**Berkhamsted Hertfordshire (GB)**
Inventor: **Robinson, John Edward**
**The Wellcome Research Laboratories**
**Berkhamsted Hertfordshire (GB)**

(74) Representative: **Bassett, Richard Simon et al**
**ERIC POTTER & CLARKSON 14 Oxford Street**
**Nottingham NG1 5BP (GB)**

**EP 0 194 764 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

(56) References cited:

TETRAHEDRON LETTERS, vol. 25, no. 38, 1984, pages 4267-4270, Pergamon Press Ltd., Oxford, GB; L. CROMBIE et al.: "Insecticidal amides. Synthesis of natural 2(E),4(E),10(E)-Pipercide, its 2(E),4(E),10(Z)-stereomer, and related isobutylamides"

CHEMICAL ABSTRACTS, vol. 98, no. 21, 23rd May 1983, page 620, no. 178980f, Columbus, Ohio, US; & JP - A - 57 212 150 (SUMITOMO CHEMICAL CO., LTD.) 27-12-1982

BER., vol. 96, 1963, pages 3349-3358, Berlin, DE; E. WINTERFELDT: "Strukturaufklärung und Synthese einer Thiophenverbindung aus Chrysanthemum frutescens L."

CHEMICAL ABSTRACTS, vol. 65, no. 4, 15th August 1966, no. 5393g, Columbus, Ohio, US; A. MEISTERS et al.: "The isobutylamides of 7-phenylhepta-2,4-dienoic acid, 7-phenylhepta-2,4,6-trienoic acid and p-(2-phenylethyl)benzoic acid", & AUSTRALIAN J. CHEM. 19(7), 1215-20, 1966

**Description**

This invention relates to pesticidal compounds.

European patent publication No. 111 015 discloses a class of w-phenyl unsaturated amide compounds as insecticides, all of these compounds having an unsaturated bond conjugated to the phenyl ring. The same document also discloses a larger class of related compounds, stated to have any 5- or 6-membered aromatic ring in the w-position and without being limited to compound having an unsaturated bond conjugated to the aromatic ring. However, these compounds are disclosed only as acaricides. We have now found that insecticidal activity is found in compounds without conjugation to the aromatic group, and also that pesticidal activity is found in compounds having aromatic or partially aromatic groups with more than 5 or 6 members.

Meisters and Wailes (Aust. J. Chem. (1966), *19*, 1215) disclose N-isobutyl (2E,4E)-7-phenylhepta-2,4-dienamide but assert that it had no insecticidal activity against *Musca domestica* at up to 100% concentration. Vig *et al* (J. Indian Chem. Soc. 1974, 51(9), 817) disclose piperovatine (N-isobutyl 6-(4-methoxyphenyl)-hexa-2,4-dienamide) but do not mention any insecticidal activity.

Japanese patent application No. 57/212,150 discloses certain w-phenyl deca-, undeca- and dodecadienamides as insecticides.

European patent publication No. 143 593, having an earliest priority date of 21st November 1983 and a publication date of 6th June 1985, discloses unsaturated amide pesticides having an w-aromatic group which is polynuclear. All of the compounds which are specifically disclosed have polynuclear systems which are entirely aromatic.

B. G. Pring, J. Chem. Soc. (Perkins Transac. 1), 1982, 1493—1498 disclosed various unsaturated amide compounds, such as pipercallosine and pipercallosidine, but did not ascribe any pesticidal activity to them. L. Crombie and R. Denman, Tet. Lett. *25* (38), 1984, 4267—4270 disclosed a number of w-aromatic unsaturated amide compounds having 3,4-methylenedioxy substitution on the phenyl ring and having six carbon atoms between the phenyl ring and the diene unsaturated moiety. Finally, E. Winterfeldt, Ber. *96*, 1963, 3349—3358 disclosed some unsaturated amides, but without ascribing any pesticidal activity to them.

This invention provides compounds of Formula (I):

$$Ar(CH_2)_n(CR^2{=}CR^3)_2C(O)NRR^1 \qquad (I)$$

wherein: Ar is phenyl, naphthyl, thienyl, fluorenyl, phenanthrenyl, dibenzofuranyl or a polynuclear group (A):

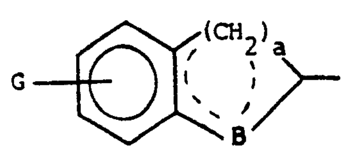

in which a is 0, 1 or 2; B is $(D)_b(CH_2)_c(E)_e$ where each of D and E is oxygen or sulphur, b and e are independently 0 or 1 but not both 1, and c is 0, 1, 2 or 3, the sum of a, b, c and e being at least 2, and the ring containing B is wholly or partially saturated; and G is hydrogen or a benzene ring fused to the benzene ring of group (A);

any of the groups Ar may be substituted by one or more of $C_{1-4}$ alkyl, halo-($C_{1-4}$) alkyl, halo, $C_{1-4}$ alkoxy (except 3,4-methylenedioxy) and halo-($C_{1-4}$-alkoxy); n is 1 to 8, except that n is 1 to 4 when Ar is phenyl or substituted phenyl; each of $R^2$ and $R^3$ is in each case independently hydrogen, $C_{1-4}$ alkyl or halo-($C_{1-4}$) alkyl; and R and $R^1$ are each selected from hydrogen, $C_{1-6}$ alkyl, $C_{3-6}$ cycloalkyl, $C_{2-6}$ alkenyl or $C_{1-6}$ alkoxy (any of which may be substituted by halo, $C_{2-6}$ alkenyl, $C_{1-6}$ alkyl, $C_{3-6}$ cycloalkyl, $C_{1-6}$ alkoxy, $C_{2-6}$ alkynyl or cyano) except for the following compounds:

N-isobutyl 7-phenyl hepta-(2E,4E)-dienamide
N-isobutyl 6-phenyl hexa-(2E,4E)-dienamide
N-isobutyl 6-(4-methoxyphenyl)hexa-(2E,4E)-dienamide
N-isobutyl 6-(2-thienyl)hexa-(2E,4E)-dienamide
N-isobutyl 8-phenylocta-(2E,4E)-dienamide
N-isobutyl 6-(1-naphthyl)hexa-(2E,4E)-dienamide

In Formula I, the following features and any and all combinations thereof are preferred:

Preferably, n is odd. Suitably, n is 1, 3 or 5, most preferably 1 or 3. The configuration of both double bonds in the diene group is preferably *E*.

Preferably groups for Ar include phenyl, furyl, thienyl, naphthyl (especially 2-naphthyl), benzofuranyl, benzopyranyl, chromanyl, indanyl, tetrahydronaphthyl, or any of the following groups:

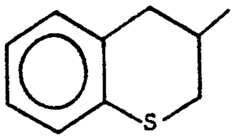
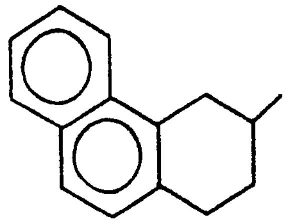

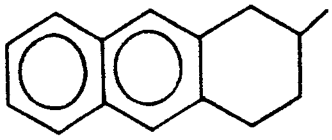
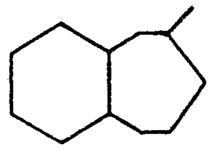

any of which may be substituted as above.

Any substitution of a single phenyl ring is preferably at the 3-position and is preferably halo (e.g. fluoro), haloalkyl (e.g. trifluoromethyl) or alkoxy (e.g. methoxy). 3,4-Dihalophenyl is also a preferred value for Ar. When Ar is naphthyl or benzofuranyl, the said $(CH_2)_n$ link is preferably attached at the 1-position.

Trifluoromethyl is particularly preferred. Preferably Ar is 3-trifluoromethylphenyl, particularly when n is 1.

Preferably, R is hydrogen. Suitably, $R^1$ is alkyl, $C_{1-6}$ being preferred isobutyl 1-methylpropyl, 2,2-dimethylpropyl, 1,2,2-trimethylpropyl and 1,2-dimethylpropyl being particularly preferable. It has been found that acaricidal activity is enhanced if there is an alkyl group α to the nitrogen.

$R^2$ and $R^3$ are preferably in each case hydrogen.

Compounds of Formula (I), (IA) and (IB) may be prepared in any of the following ways:—

(a) by amidation of the corresponding acid or acid derivative, i.e. by reaction of a compound of Formula (II) with a compound of Formula (III):

$$Ar(CH_2)_n(CR^2{=}CR^3)_2\ COZ' \qquad (II)$$

$$HNRR^1 \qquad (III)$$

wherein $Z^1$ is hydroxyl, halo or a phosphoroimidate ester group

$O^-$
|
(—P(O Aryl)NH aryl)

and the other variables are as defined above;

(b) by reaction of a compound of Formula (IV) with a compound of Formula (V) or (VI);

O
||
$Ar(CH_2)_n(CR^2{=}CR^3)_pCH$ (IV)

$$(Z'')_3P{=}CH(CR^2{=}CHR^3)_qC(O)NRR^1 \qquad (V)$$

$(Z'')_2P{=}CH(CR^2{=}CR^3)_qC(O)NRR^1$
|
$O^-$ (VI)

wherein Z″ is alkyl, alkoxy (preferably ethoxy) or aryl (preferably phenyl), and p + q = 1. The locations of the aldehyde and the phosphorus containing groups, $(Z'')_3P$ and $(Z'')_2P(O)$, may be swapped to give an exactly analogous reaction;

(c) by β-elimination from a compound of Formula (VII) or (VIII):

X Y
| |
$Ar(CH_2)_n(CR^2{=}CR^3)CR^2CR^3C(O)NRR^1$ (VII)

X Y
| |
$Ar(CH_2)_nCR^2CR^3(CR^2{=}CR^3)C(O)NRR^1$ (VIII)

wherein one of X and Y is hydrogen and the other is a group $Q(\rightarrow O^-)L$, Q is sulphur or selenium and L is a suitable group such as lower alkyl (preferably methyl) or aryl;

(d) by reaction of a compound of Formula (IX) with a compound of Formula (X):—

$$Ar(CH_2)_nCH_2\text{-}Hal \qquad (IX)$$

$$R^2C{\equiv}CR^3(CR^2{=}CR^3)C(O)NR^1 \qquad (X)$$

where Hal is a halogen atom, followed by reduction of the triple bond; or

(e) by reacting a compound of Formula (XI) with a compound of Formula (XII):

$$Ar(CH_2)_nCR^2{=}CR^3{-}M \qquad (XI)$$

O
‖
$Hal{-}(CR^2{=}CR^3)CNRR^1$ (XII)

wherein Hal is halide e.g. bromide or iodide and M is a metal atom or metal group, for example comprising zirconium, aluminium or zinc, e.g. a bis-(cyclopentadienyl) zirconium chloride group. Compounds of Formula (XI) may be made with, for example, vinyl-bis-(cyclopentadienyl)zirconium chloride in THF in the presence of a palladium (O) catalyst.

Process (a) is normally carried out in an aprotic solvent, such as ether, dichloromethane or benzene, optionally in the presence of a tertiary amine such as triethylamine, but in the absence of water. If the compound of Formula (I) is an acid halide, for example acid chloride, then it may be formed from the corresponding acid by reaction with a suitable reagent such as oxalyl chloride or thionyl chloride. When Z′ is a phosphoroimidate group then this is suitably formed from $(PhO)P(\rightarrow O)NHPh$ Cl. The acid, or the acid function in the compound of Formula (II), may be prepared by hydrolysis of an ester, the ester being prepared by a conventional Wittig or Wadsworth-Emmons reacton, using for example an aldehyde and ethoxycarbonylmethylene triphenylphosphorane or the anion from triethylphosphonocrotonate. This latter reaction may result in an isomeric mixture, for example a (2,4)-hexadienoate and (3,5)-hexadienoate; such a mixture may be reacted as above, and the resulting mixture of amides separated by chromatography or other convenient techniques. When n is 1, hydrolysis of the ester is preferably acidic, for example using aqueous hydrochloric acid and dioxan.

Alternatively, the ester referred to above may be derived by rearrangement and elimination on a compound of Formula (XIII):

$SR^5$
/
$Ar(CH_2)_{n+2}CR^2{=}C$ (XIII)
\
$COOR^4$

wherein $R^5$ is any suitable group, such as phenyl, and $R^4$ is alkyl.

The compound of Formula (XIII) may be obtained by reaction of a compound of Formula (XIV) with a compound of Formula (XV):

O
‖
$Ar(CH_2)_{n+2}CH$ (XIV)

O
‖
$PhS(\rightarrow O)CH_2COR^4$ (XV)

A further route is for the ester referred to above to be prepared by elimination on a compound of formula (XVI):

$$Ar(CH_2)_n{-}A^1{-}A^2{-}A^3{-}COOR^4 \qquad (XVI)$$

wherein $R^4$ is as defined above, one of $A^1$, $A^2$ and $A^3$ is $(CR^2{=}CR^3)$, another of $A^1$, $A^2$ and $A^3$ is $-CR^2R^3-$, the third of $A^1$, $A^2$ and $A^3$ is $-CR^2(OR^6)-$, $R^6$ being H or acyl such as acetyl, and the said $-CR^2R^3-$ and $-CR^2(OR^6)-$ groups are adjacent one another. The reaction is preferably carried out in an aromatic solvent, conveniently in the presence of a molybdenum catalyst and a base, such as bis-trimethylsilylacetamide.

Intermediates of Formula (XVI) may be obtained by reaction of a suitable aldehyde with a suitable sulphinyl compound, followed by acylation.

The reaction is carried out in a suitable solvent such as acetonitrile with a base such as piperidine.

Process (b) is carried out in a dry solvent, for example tetrahydrofuran, optionally in the presence of a

base, and preferably in the absence of oxygen, e.g. under a nitrogen atmosphere, at a low temperature. The Wittig-type reagent may be obtained with lithium diisopropylamide.

Process (c) is normally carried out by heating in an aprotic solvent such as benzene or toluene, preferably in the presence of an acid catlayst, such as paratoluene-sulphonic acid. Process (d) proceeds by reaction of the compound of formula (X) with a base (such as lithium diisopropylamide) and the compound of Formula (IX) in an aprotic solvent such as THF. Process (e) is preferably carried out in an aprotic solvent such as THF, under an inert atmosphere (such as argon) and in the presence of a palladium (O) catalyst, such as bis(triphenylphosphine)palladium.

The intermediates of Formulae (III)—(XIV) may be prepared by standard methods. For example, the compounds of Formulae (V) and (VI) may be prepared by the reaction of an appropriate phosphine, phosphonate or phosphite with an w-halo amide. Compounds of Formula (IV) may be prepared by hydrolysis of a ketal ring or oxidation of an alcohol.

The carbonyl-containing compounds of Formula (IV) may be prepared by oxidation of the corresponding alcohol, for example using pyridinium chlorochromate or oxalyl chloride/DMSO.

The compounds of Formula (I) may be used to control arthropods such as insect and acarine pests.

The compounds of Formula (I) may be used for such purposes by application of the compounds themselves or in diluted form in known fashion as a dip, spray, lacquer, foam, dust, powder aqueous suspension, paste, gel, shampoo, grease, combustible solid, vapourising mat, wettable powder, granule, aerosol, emulsifiable concentrate, oil suspensions, oil solutions, pressure-pack, impregnated article (such as an ear tag or collar) or pour on formulation. Dip concentrates are not applied *per se*, but diluted with water and the animals immersed in a dipping bath containing the dip wash. Sprays may be applied by hand or by means of a spray race or arch. The animal may be saturated with the spray by means of high volume application or superficially coated with the spray by means of light or ultra low volume application. Aqueous suspensions may be applied to the animal in the same manner as sprays or dips. Dusts may be distributed over the animals by means of a powder applicator or incorporated in perforated bags attached to trees or rubbing bars. Pastes, shampoos and greases may be applied manually or distributed over the surface of an inert material against which animals rub and transfer the material to their skins. Pour-on formulations are dispensed as a unit of liquid of small volume on to the backs of animals such that all or most of the liquid is retained on the animals.

The compounds of formula (I) may be formulated either as formulations ready for use on the animals or as formulations requiring dilution prior to application, but both types of formulation comprise a compound of formula (I) in intimate admixture with one or more carriers or diluents. The carriers may be liquid solid or gaseous or comprise mixtures of such substances, and the compound of formula (I) may be present in a concentration of from 0.025 to 99% w/v depending upon whether the formulation requires further dilution.

Dusts, powder and granules comprise the compound of formula (I) in intimate admixture with a powdered solid inert carrier for example suitable clays, kaolin, talc, mica, chalk, gypsum, vegetable carriers, starch and diatomaceous earths.

Sprays of a compound of formula (I) may comprise a solution in an organic solvent (e.g. those listed below) or an emulsion in water (dip wash or spray wash) prepared in the field from an emulsifiable concentrate (otherwise known as a water miscible oil) which may also be used for dipping purposes. The concentrate preferably comprises a mixture of the active ingredient, with or without an organic solvent and one or more emulsifiers. Solvents may be present within wide limits but preferably in an amount of from 0 to 90% w/v of the composition and may be selected from kerosene, ketones, alcohols, xylene, aromatic naphtha, and oher solvents known in the formulating art. The concentraton of emulsifiers may be varied within wide limits but is preferably in the range of 5 to 25% w/v and the emulsifiers are conveniently nonionic surface active agents including polyoxyalkylene esters of alkyl phenols and polyoxyethylene derivatives of hexitol anhydrides and anionic surface active agents including Na lauryl sulphate, fatty alcohol ether sulphates, Na and Ca salts of alkyl aryl sulphonates and alkyl sulphosuccinates.

Wettable powders comprise an inert solid carrier, one or more surface active agents, and optionally stabilisers and/or anti-oxidants.

Emulsifiable concentrates comprise emulsifying agents, and often an organic solvent, such as kerosene, ketones, alcohols, xylenes, aromatic naphtha, and other solvents known in the art.

Wettable powders and emulsifiable concentrates will normally contain from 5 to 95% by weight of the active ingredient, and are diluted, for example with water, before use.

Lacquers comprise a solution of the active ingredient in an organic solvent, together with a resin, and optionally a plasticiser.

Dip washes may be prepared not only from emulsifiable concentrates but also from wettable powders, soap based dips and aqueous suspensions comprising a compound of formula (I) in intimate admixture with a dispersing agent and one or more surface active agents.

Aqueous suspensions of a compound of formula (I) may comprise a suspension in water together with suspending, stabilizing or other agents. Aqueous solutions may also be formed from acid addition salts of a compound of the formula (I). The suspensions or solutions may be applied *per se* or in a diluted form in known fashion.

Greases (or ointments) may be prepared from vegetable oils, synthetic esters of fatty acids or wool fat

together with an inert base such as soft paraffin. A compound of formula (I) is preferably distributed uniformly through the mixture in solution or suspension. Greases may also be made from emulsifiable concentrates by diluting them with an ointment base.

Pastes and shampoos are also semi-solid preparations in which a compound of formula (I) may be present as a uniform dispersion in a suitable base such as soft or liquid paraffin or made on a non-greasy basis with glycerin, mucilage or a suitable soap. As greases, shampoos and pastes are usually applied without further dilution they should contain the appropriate percentage of the compound of formula (I) required for treatment.

Aerosol sprays may be prepared as a simple solution of the active ingredient in the aerosol propellant and co-solvent such as halogenated alkanes and the solvents referred to above, respectively. Pour-on formulations may be made as a solution or suspension of a compound of formula (I) in a liquid medium which aso contains a viscous oil to minimise spreading of the formulation on the surface of the animals. An avian or mammal host may also be protected against infestation of Acarine ectoparasites by means of carrying a suitably-moulded, shaped plastics article impreganted with a compound of formula (I). Such articles include impregnated collars, tags, bands, sheets and strips suitably attached to appropriate parts of the body.

The concentration of the compound of formula (I) to be applied to an animal will vary according to the compound chosen, the interval between treatments, the nature of the formulation and the likely infestation, but in general 0.001 to 20.0% w/v and preferably 0.01 to 10% of the compound should be present in the applied formulation. The amount of the compound deposited on an animal will vary according to the method of application, size of the animal, concentration of the compound in the applied formulation, factor by which the formulation is diluted and the nature of the formulation but in general will lie in the range of from 0.0001% to 0.5% except for undiluted formulations such as pour-on formulations which in general will be deposited at a concentration in the range from 0.1 to 20.0% and preferably 0.1 to 10%.

Dusts, greases, pastes and aerosol formulations are usually applied in a random fashion as described above and concentrations of 0.001 to 20% w/v of a compound of formula (I) in the applied formulation may be used.

Insect pests include members of the orders Coleoptera (e.g. *Anobium, Tribolium, Sitophilus, Diabrotica, Anthonomus* or *Anthrenus* spp.), Lepidoptera (e.g. *Ephestia, Plutella, Chilo, Heliothis, Spodoptra* or *Tineola* spp.), Diptera (e.g. *Musca, Aedes, Culex, Glossina, Stomoxys, Haematobia, Tabanus, Hydrotaea, Lucilia, Chrysomia, Callitroga, Dermatobia, Hypoderma, Liriomyza,* and *Melophagus* spp.), Phthiraptera (*Malophaga* e.g. *Damalina* spp. and *Anoplura* e.g. *Linognathus* and *Haematopinus* spp.), Hemiptera (e.g. *Aphis, Bemisia, Aleurodes, Nilopavata, Nephrotetix* or *Cimex* spp.), Orthoptera (e.g. *Schistocerca* or *Acheta* spp.), Dictyoptera (e.g. *Blattella, Periplaneta* or *Blatta* spp.), Hymenoptera (e.g. *Solenopsis* or *Monomorium* spp.), Isoptera (e.g. *Reticulitermes* spp.), Siphonaptera (e.g. *Ctenocephalides* or *Pulex* spp.), Thysanura (e.g. *Lepisma* spp.), Dermaptera (e.g. *Forficula* spp.) and Pscoptera (e.g. *Peripsocus* spp.).

Acarine pests include ticks, e.g. members of the genera *Boophilus, Rhipicephalus, Amblyomma, Hyalomma, Ixodes, Haemaphysalis, Dermocentor* and *Anocentor*, and mites and manges such as *Tetranychus, Psoroptes, Psorergates, Chorioptes* and *Demodex* spp.

The compounds exhibit killing and/or knockdown activity against arthropod pests, and can be used to control larval pests as well as adult pests.

Compounds of the invention may be combined with one or more other active ingredients (for example pyrethroids, carbamates and organophosphates) and/or with attractants and the like. Furthermore, it has been found that the activity of the compounds of the invention may be enhanced by the addition of a synergist or potentiator, for example: one of the oxidase inhibitor class of synergists, such as piperonyl butoxide or NIA 16388; a second compound of the invention; or a pyrethroid pesticidal compound. When an oxidase inhibitor synergist is present in a formula of the invention, the ratio of synergist to compound of formula (I) will be in the range 25:1—1:25 e.g. about 10:1.

Stabilisers for preventing any chemical degradation which may occur with the compounds of the invention include, for example, antioxidants (such as tocopherols, butylhydroxyanisole and butylhydroxytoluene) and scavengers (such as epichlorhydrin).

It will be understood that what we will claim may comprise:

(a) compounds of Formula (I);

(b) processes for the preparation of compounds of Formula (I);

(c) insecticidal and acaricidal compositions comprising a compound of Formula (I) in admixture with a carrier;

(d) processes for the preparation of such pesticidal compositions;

(e) methods for the control of insect or acarine pests comprising the application to the pest or its environment of a compound of Formula (I);

(f) synergised pesticidal compositions comprising a compound of Formula (I);

(g) potentiating or non-potentiating mixtures of a compound of Formula (I) and another pesticidal compound; and

(h) novel intermediates of the preparation of compounds of Formula (I), especially compounds of Formula (II) and (XI).

The following Examples illustrate, in a non-limiting manner, preferred embodiments of the invention.

Example 1

(2E),(4E)-N-Isobutyl 6-phenyl-2,4-hexadienamide (not claimed *per se*)

Triethylphosphonocrotonate (20.85 g, 83 mmol) in tetrahydrofuran (THF) (50 ml) was added at −70°C to lithium diisopropylamide (83 mmol) in THF (50 ml). The temperature of the mixture was allowed to reach −20°C and recooled to −40°C. Phenylacetaldehyde (10 g, 83 mmol) in THF (30 ml) was added. The mixture was left overnight at room temperature and worked up in the standard manner. The crude material was purified by column chromatography (silica; 9:1 hexane:ether) to give a yellow oil (10 g, 56%) consisting substantially of (2*E*,4*E*) ethyl 6-phenyl-2,4-hexadienoate and the 3,5-dienoate.

The mixture of esters (10 g), water (40 ml), conc. HCl (60 ml) and dioxan (200 ml) was heated under reflux for 16 h. The product was extracted into ether, washed with $NaHCO_3$ and brine, and dried. Solvents were removed to give a crude product, (2*E*),(4*E*)-6-phenyl-2,4-hexadienoic acid and (3*E*,5*E*)-6-phenyl-3,5-hexadienoic acid (B), which was carried on to the next stage.

Triethylamine (6.4 ml, 46.3 mmol) was added to the acids (B) (8.7 g, 46.3 mmol) and phenyl *N*-phenylphosporamidochloridate (12.4 g, 46.3 mmol) in dichloromethane (about 50 ml) with cooling. The yellow solution was stirred under nitrogen for 1 hr at room temperature, and then triethylamine (6.4 ml, 46.3 mmol) and isobutylamine (4.6 ml, 46.3 ml) were added with cooling. After 16 hr at room temperature the reaction was worked up with ether. The crude title product was purified, first by column chromatography (silica hexane/ether) then by recrystallisation (6:4 hexane:ethyl acetate) to give colourless needles (1.7 g), m.p. 119—20°C. Tlc. Silica/ether, 1 spot Rf 0.46, GC (OV210 200°); retention time 1.3 min.

NMR: 7.16, (6H), m, aryl, H3; 6.20 (3H), m, H4, H5, NH; 5.83 (1H), d, H2; 3.48 (2H), d, H6; 3.16 (2H), d of d, 1.8 (1H), m, 0.95 (6H), d, isobutyl.

Example 2

(2E),(4E)-N-isobutyl 6-(3-trifluoromethylphenyl)-2,4-hexadienamide

3-Trifluoromethylbromobenzene (3 g, 13.3 mmol), prop-2-yne-1-ol (13.3 mmol) *bis*-triphenylphosphine palladium chloride (0.2 g) and cuprous iodide (80 mg) in triethylamine (20 ml) were treated together at room temperature under dry nitrogen for 16 hrs. The reaction was worked up and the crude product purified by column chromatography (silica/ether)(3.4 g).

Compound (A) (3.4 g), 17 mmol was subjected to catalytic hydrogenation in ethyl acetate solution in the presence of 5% palladium on charcoal. Hydrogen (820 ml) was taken up, the solution filtered and the solvent removed under reduced pressure to give 3-(3-trifluoromethylphenyl)-propan-1-ol (B) (3.5 g), which was used without further purification.

Redistilled oxalyl chloride (1.6 ml, 18.33 mmol) in dry dichloromethane (30 ml) was cooled to −60°C. Dimethyl sulphoxide (2.85 g, 36.6 mmol) was added and, after 10 mins, the alcohol (B) (3.4 g, 16.7 mmol) was added. The temperature was held at −60°C for 1.5 hrs, and then triethylamine (11.6 ml, 84 mmol) was added. The mixture was allowed to reach room temperature and extracted into dichloromethane with washing by aq. HCl, $NaHCO_3$ and brine. The solution was dried and the solvents removed to give 3-(3-trifluoromethylphenyl)-propan-1-al (C) which was used without further purification.

To methyl triphenylphosphonium iodide (5 g, 12.4 mmol) in dry THF (30 ml) was added n-butyl lithium (7.8 ml at 1.6M, 12.4 mmol) at −20°C. After 1 hr, the aldehyde (C) (2.5 g, 12.4 mmol) in THF was added. After 18 hrs at room temperature under nitrogen, the reaction mixture was worked-up in conventional fashion. the crude material was purified by column chromatography (Silica/hexane) to give, 4-(3′-trifluoromethylphenyl)-but-1-ene (D) (2 g, 80%).

To *N*-isobutyl methylsulphinylacetamide (1.55 g, 8.75 mmol) in trifluoroacetic acid (12 ml) under nitrogen at 0°C, was added trifluoroacetic anhydride (1.23 ml, 8.75 mmol). After 10 mins the olefin (D) (1.75 g, 8.75 mmol) was added. The reaction mixture was left overnight at room temperature and worked-up in conventional fashion. The crude material was purified by column chromatography (Silica, hexane/ether, 1:1) to give (*E*)-*N*-isobutyl 6-(3′-trifluoromethylphenyl)-2-methylthio-4-hexenamide (E) (1.06 g, 32%).

Compound E (1 g, 2.79 mmol) in methanol was treated with sodium periodate (0.57 g, 2.65 mmol) in water (18 ml) at 0°C. The mixture was left overnight and then filtered, and the filtrate was extracted with chloroform. The organic extracts were dried and the solvent removed. The crude sulphoxide was dissolved in dry toluene (20 ml) and heated under reflux for 10 hrs. After a standard work-up, flash-column chromatography (silica, 4:1 ether:hexane) gave the title product as a colourless solid (0.2 g, 24%), m.p. 133—135°C. Tlc (silica/ether) 1 spot Rf 0.43. Glc (3% OV210 200°) 1 peak. Retention time 1.5 mins.

NMR spectrum: 7.40 (5H), m, aromatic, H3; 6.08 (3H), m, H4, H5, NH; 5.83 (1H), d, H2; 3.55 (2H), m, H6; 3.18 (2H), d of d, 1.8 (1H) m, 0.95 (6H), d, isobutyl.

Example 3

(2E),(4E)-N-isobutyl 6-(3′-fluorophenyl)-2,4-hexadienamide

4-(3-fluorophenyl)-but-3-yne-1-ol (A) was prepared from but-3-yne-1-ol and 3-fluoro-iodobenzene used an analogous sequence to that of Example 2 and was then similarly reacted to give 4-(3-fluorophenyl)-

butan-1-ol (B), which in turn gave 4-(3-fluorophenyl)-butan-1-al (C) as in Example 2.

Compound (C) (0.8 g, 4.8 mmol), methyl 2-phenylsulphinylacetate (0.61 g, 3.2 mmol) and piperidine (0.027 g, 0.32 mmol) were reacted in dry acetonitrile (15 ml) under nitrogen at 0°C for 2 days. Working up gave a crude product which was purified by column chromatography (silica, ether/hexane) to give methyl 2-phenylsulphinyl-6-(3-fluorophenyl)-2-hexenoate (D).

Potassium carbonate (anhydrous, 0.163 g, 1.18 mmol) was added to a solution of (D) (0.34 g, 0.98 mmol) in dry xylene (3 ml). After heating to reflux, the mixture was cooled and the solvent evaporated to give a crude product. Flash column chromatography (silica, ether/hexane) gave methyl 6-(3-fluorophenyl)-2,4-hexadienoate, (170 mg).

This was treated by the method of Example 1 to give 6-(3-fluorophenyl)-2,4-hexadienoic acid (F), which, also as in Example 1, yielded the title compound (20 mg).

Off-white solid m.p. 108—11°; Tlc (silica/ether), 1 spot, Rf 0.38; Glc (3% Ov210 220°), Retention time 1.0 min.

NMR: 7.22 (1H), m, H3; 6.90 (4H), m, aromatic; 6.15 (2H), m, H4, H5; 5.78 (H), d, H2; 5.43 (1H), bd, NH; 3.50, (2H), d, H6; 3.16 (2H), d of d, 1.8 (1H), m, 0.92, (6H) d, isobutyl.

## Example 5

(2E),(4E)-N-Isobutyl 8-(3-trifluoromethylphenyl)-2,4-octadienamide

3-Trifluoromethylbromobenzene (4 g, 17.8 mmol) and but-3-yne-1-ol (1.24 g 17.8 mmol) were reacted together in the presence of bis-triphenylphosphine palladim dichloride-cuprous iodide, as in Example 2, to give 4-(3-trifluoromethylphenyl)-but-3-yne-1-ol (A) which was subjected to hydrogenation, as in Example 2, to give 4-(3-trifluoromethylphenyl)-butan-1-ol (B). Compound (B) was oxidised, as in Example 2, to give 4-(3-trifluoromethylphenyl)-butan-1-al (C) which (2.2 g, 10.18 mmol) was reacted with triethylphosphonocrotonate-lithium diisopropylamide, as in Example 1, to give ethyl 8-(3-trifluoromethylphenyl)-2,4-octadienoate (D).

Compound (D) (1.7 g, 5.45 mmol) was hydrolysed with potassium hydroxide (1.67 g, 19.07 mmol) in aqueous ethanol at room temperature, under nitrogen. The usual work up afforded 8-(3-trifluoromethylphenyl)-2,4-octadienoic acid (E). Compound (E) was reacted with N-phenylphosphoramidochloridate, triethylamine and isobutylamine as in Example 1, to give (2E),(4E)-N-isobutyl 8-(3-trifluoromethylphenyl)-2,4-octadienamide as a pale yellow solid (230 mg). Tlc (silica/ether), 1 spot Rf 0.44, GC (OV210,230°); retention time 1.0 min.

NMR: 7.39 (4H), m, aryl; 7.20 (1H), m, H3; 6.10 (2H), m, H4, H5; 5.81 (1H), d, H2; 5.8 (1H), bd, NH; 2.75 (2H), t, H8; 1.95 (4H), m, H6, H7; 3.20 (2H), d of d, 0.95 (6H), d, isobutyl.

## Example 6

(2E),(4E)-N-isobutyl 6-(2-naphthyl)-2,4-hexadienamide

2-Bromonaphthalene (10 g, 48.3 mmol) and but-3-yne-1-ol (3.38 g, 48.3 mmol) were reacted together, analogously to Example 2, to give 4-(2-naphthyl)-but-3-yne-1-ol (A) which was hydrogenated, as in Example 2, to give 4-(2-napthyl)-butan-1-ol, oxidised to 4-(2-napthyl)-butan-1-al (C). Compound (C) (1.6 g, 8.08 mmol) in dry acetonitrile was added over 1.5 h to a solution of methyl 2-(4-chlorophenyl)sulphinyl acetate (1.78 g, 7.68 mmol) and piperidine (0.69 g, 8.08 mmol) in dry acetonitrile. After 16 h at room temperature, under nitrogen, the reaction was worked up to give, after column chromatography (silica, 8:2 ether:hexane), methyl 4-hydroxy-6-(2-napthyl)-2-hexenoate (1.32 g) (D).

Compound (D) (1.32 g, 4.89 mmol), acetic anhydride (1.05 g, 10.27 mmol) and triethylamine (0.74 g, 7.34 mmol) were treated with 4-N,N-dimethylaminopyridine (50 mg) at 0°C. After several hours at room temperature, the reaction mixture was worked up and the crude acetate was purified on a silica column to give methyl 4-acetoxy-6-(2-napthyl)-2-hexenoate (1.2 g) (E).

Compound (E) (1.2 g, 4.05 mmol) in dry toluene (10 ml) was heated under reflux under dry nitrogen, with bis-trimethylsilylacetamide (0.82 g, 4.05 mmol) and molybdenum hexacarbonyl (0.84 g, 3.2 mmol) for about 2 h. The reaction was worked up and the product purified by flash column chromatography to give methyl-6-(2-napthyl)-2,4-hexadienoate (0.35 g) which was hydrolysed, as in Example 1, to give the acid, in turn converted to (2*E*),(4*E*) *N*-isobutyl 6-(2-naphthyl)-2,4-hexadienamide (120 mg), m.p. 138—40°; Tlc, silica/ether, Rf 0.40.

NMR: 7.6 (7H), m, aryl; 7.25 (1H), m, H3; 6.25 (2H), m, H4, H5; 5.77 (1H), d, H2; 5.45 (1H), bd, NH; 3.66 (2H), d, H6; 3.18 (2H), d of d, 1.80 (1H), m, 0.93 (6H), d, isobutyl.

## Example 7

(2E),(4E)-N-Isobutyl 8-(2-benzofuranyl)-2,4-octadienamide

1-Iodophenol (6 g 27.4 mmol) and but-3-yne-1-ol (2.68 g, 27.4 mmol) were reacted together over a prolonged period with bis-triphenyl-phosphine palladium dichloride-cuprous iodide in diethylamine to yield 4-(2-benzofuranyl)-butan-1-ol (A).

This compound was taken through to the title compound using a sequence analogous to that in Example 5, yielding 0.43 g. GC (OV210 at 250°), retention time 1.5 mins.

NMR: 7.20, (5H), m, aryl, H3; 6.37 (1H), s, benzofuran H3; 6.18 (2H), m, H4, H5; 5.78 (1H), d, H2; 5.6 (1H), bd, NH; 2.08 (4H), m, H6, H7; 2.78 (2H), t, H8; 3.21 (2H), d of d, 0.95 (6H), d, isobutyl.

Examples 8 to 89

By analogous methods, the compounds listed in Table 1 and Table 2 were made. In all the compounds, the configuration of the double bonds conjugated to the amide carbonyl was *E*.

TABLE 1

$Ar(CH_2)_n(CH{=}CH)_mCONHR$

| Example No. | Ar | n | m | R | Preparation as Example No. |
|---|---|---|---|---|---|
| 8. | Ph | 1 | 2 | 1,2-dimethylpropyl | 1 |
| 9. | Ph | 1 | 2 | 2-methoxypropyl | 1 |
| 10. | Ph | 1 | 2 | 2-methylprop-2-ene | 1 |
| 12. | Ph | 3 | 2 | isobutyl | 5 |
| 13. | Ph | 3 | 2 | sec-butyl | 5 |
| 14. | Ph | 3 | 2 | 1,2-dimethylpropyl | 5 |
| 15. | Ph | 3 | 2 | 1-cyano-2-methylpropyl | 5 |
| 16. | Ph | 3 | 2 | cyclohexyl | 5 |
| 17. | 3-Trifluoromethylphenyl | 2 | 2 | isobutyl | 5 |
| 18. | 3-Trifluoromethylphenyl | 3 | 2 | 1,2-dimethylpropyl | 5 |
| 19. | 3-trifluoromethylphenyl | 3 | 2 | 2-methylpropyl | 5 |
| 20. | 3,5-*bis*-trifluoromethylphenyl | 3 | 2 | isobutyl | 5 |
| 21. | 3,5-*bis*-trifluoromethylphenyl | 3 | 2 | 1,2-dimethylpropyl | 5 |
| 22. | 3-Fluorophenyl | 2 | 2 | isobutyl | 5 |
| 23. | 3-Fluorophenyl | 3 | 2 | isobutyl | 5 |
| 24. | 3-Fluorophenyl | 3 | 2 | 1,2-dimethylpropyl | 5 |
| 25. | 3,5-Dichlorophenyl | 3 | 2 | 1,2-dimethylpropyl | 5 |
| 26. | 3,5-Dichlorophenyl | 7 | 2 | 4-methylpentyl | 5 |
| 27. | 2-Naphthyl | 3 | 2 | isobutyl | 5 |
| 28. | 2-Naphthyl | 3 | 2 | 1,2-dimethylpropyl | 5 |
| 31. | 2-Thienyl | 3 | 2 | isobutyl | 5 |
| 32. | 2-Thienyl | 3 | 2 | 1,2-dimethylpropyl | 5 |
| 33. | 2-Benzofuranyl | 3 | 2 | 1,2-dimethylpropyl | 7 |
| 34. | 2-Benzofuranyl | 6 | 2 | isobutyl | 7 |
| 35. | 3,5-*bis*-trifluoromethylphenyl | 1 | 2 | isobutyl | 2 |
| 36. | 4-methoxyphenyl | 1 | 2 | isobutyl | 6 |
| 37. | 4-methoxyphenyl | 1 | 2 | 2-methoxypropyl | 6 |
| 38. | phenyl | 1 | 2 | 2-methyl-2-methoxypropyl | 1 |

TABLE 1 (continued)

| Example No. | Ar | n | m | R | Preparation as Example No. |
|---|---|---|---|---|---|
| 39. | 2-naphthyl | 1 | 2 | 1,2-dimethylpropyl | 6 |
| 40. | 2-naphthyl | 1 | 2 | 2-methoxypropyl | 6 |
| 41. | 3-methoxyphenyl | 1 | 2 | isobutyl | 6 |
| 42. | 3-methoxyphenyl | 1 | 2 | 1,2-dimethylpropyl | 6 |
| 43. | pentafluorophenyl | 3 | 2 | isobutyl | 5 |
| 44. | phenyl | 3 | 2 | dimethylcyclopropyl | 5 |
| 45. | phenyl | 3 | 2 | 1,2,2-trimethylpropyl | 5 |
| 46. | phenyl | 3 | 2 | 1-ethyl-propyl | 5 |
| 47. | phenyl | 3 | 2 | 3:1, R(−):S(+) 1,2-dimethylpropyl | 5 |
| 48. | phenyl | 3 | 2 | 3:1, 5(+):R(−) 1,2-dimethylpropyl | 5 |
| 49. | phenyl | 3 | 2 | 1-methoxy-2-methylpropyl | 5** |
| 50. | 3-methoxyphenyl | 3 | 2 | 1,2-dimethylpropyl | 5 |
| 51. | 4-bromophenyl | 3 | 2 | 1,2-dimethylpropyl | 5 |
| 52. | 4-bromophenyl | 3 | 2 | 1,2-dimethylpropyl | 5 |
| 53. | 4-bromophenyl | 3 | 2 | 1,2,2-trimethylpropyl | 5 |
| 54. | 3,4-dichlorophenyl | 3 | 2 | 1,2-dimethylpropyl | 5 |
| 55. | 3,4-dichlorophenyl | 3 | 2 | 1,2,2-trimethylpropyl | 5 |
| 56. | 1-Naphthyl | 4 | 2 | 1,2-dimethylpropyl | 5 |
| 57. | 1-Naphthyl | 4 | 2 | 1,2,2-trimethylpropyl | 5 |
| 58. | phenyl | 3 | 2 | 1-fluoromethyl-2-methylpropyl | 5 |
| 59. | phenyl | 3 | 2 | 1-ethynyl-2-methylpropyl | 5 |
| 60. | 3-[O(CH$_2$)OPh]Ph | 1 | 2 | isobutyl | 6 |
| 61. | 3-[O(CH$_2$)$_2$OMe]Ph | 1 | 2 | isobutyl | 6 |
| 62. | 3-[O(CH$_2$)$_2$OMe]Ph | 3 | 2 | isobutyl | 5 |
| 63. | 3-[O(CH$_2$)$_2$OMe]Ph | 3 | 2 | 1,2-dimethylpropyl | 5 |
| 64. | 2-Benzofuranyl | 5 | 2 | isobutyl | |
| 65. | 2-Benzofuranyl | 4 | 2 | isobutyl | |
| 66. | 2-Benzofuranyl | 4 | 2 | 1,2-dimethylpropyl | |

TABLE 1 (continued)

| Example No. | Ar | n | m | R | Preparation as Example No. |
|---|---|---|---|---|---|
| 67. | 2-Benzofuranyl | 4 | 2 | 2-methoxypropyl | |
| 68. | 2-Benzofuranyl | 5 | 2 | 1,2-dimethylpropyl | |
| 69. | 2-Benzofuranyl | 2 | 2 | isobutyl | |
| 70. | 2-Benzofuranyl | 2 | 2 | 1,2-dimethylpropyl | |
| 71. | Phenyl | 3 | 2 | 1-methyl-2-methoxypropyl | |
| 72. | 2-Thianaphthenyl | 6 | 2 | isobutyl | |
| 73. | 2-Thianaphthenyl | 6 | 2 | 1,2-dimethylpropyl | |
| 74. | Phenyl | 3 | 2 | [2-(1,3-dioxalano)]-methyl | |
| 75. | 1-Naphthyl | 5 | 2 | 1,2-dimethylpropyl | |
| 76. | Phenyl | 3 | 2 | 1-ethyl-2-methyl-propyl | |
| 77. | Phenyl | 3 | 2 | cyclopentylmethyl | |
| 77A. | 3-trifluoromethylphenyl | 1 | 2 | 2-methoxypropyl | |

Example 78

(2E),(4E)-*N*-isobutyl 6-(1,2,3,4-tetrahydronaphtha-2-yl)hexa-2,4-dienamide

Sodium (1.53 g) was reacted with diethylmalonate (10.99 g) in anhydrous ethanol (150 ml) and 2-bromo-1-tetralone (15 g) (prepared by standard literature methods) was added at room temperature. The reaction mixture was heated under reflux for 2 hours under nitrogen. The ethanol was removed, the residue diluted with water and the aqueous mixture partitioned with ether and worked up in standard fashion to give an oil.

The product from above was dissolved in glacial acetic acid (225 ml) and treated with conc. hydrochloric acid (225 ml) and water (90 ml). After heating under reflux for 10 hours and allowing to cool, the mixture was partitioned between water and ether and worked up in standard fashion to give crude product acid.

The above acid (13 g) in toluene (100 ml) was treated with amalgamated zinc, prepared from zinc powder (100 g) and mercurous chloride (10 g). The mixture was heated to reflux and conc. hydrochloric acid (140 ml) added in 2 portions. After heating under reflux for 7 hours, the mixture was worked up in

Sodium borohydride (1.63 g) in dry tetrahydrofuran (70 ml) was added to the above acid (7.6 g) in dry THF (20 ml). After 10 mins boron trifluoride etherate (8.52 g) was added and the whole stirred for 18 hrs at room temperature under nitrogen. The reaction mixture was poured onto ice-hydrochloric acid and worked up in the standard manner to give a crude product which was purified by column chromatography to give 1,2,3,4-tetrahydronaphtha-2-yl ethanol.

The previous alcohol (4.3 g) was subjected to Swern oxidation as described previously using oxalyl chloride (2.35 ml), dimethylsulphoxide (3.8 ml) and triethylamine (16.95 ml) in dichloromethane to give 1,2,3,4-tetrahydronaphtha-2-yl ethanal.

The aldehyde (4 g) was treated with the anion derived from triethyl phosphonocrotonate (5.75 g) and lithium diisopropylamide (22.99 mmol) in tetrahydrofuran as in previous examples. The crude product was purified by column chromatography (silica:ethyl-hexane) to give an ester (3.65 g) which was hydrolysed by aqueous hydrochloric acid/dioxane to give 6-(1,2,3,4-tetrahydronaphtha-2-yl)hexa-2,4-dienoic acid.

The above acid (0.4 g) was reacted with phenyl *N*-phenylphosphoramidochloridate (0.44 g), isobutylamine (0.165 ml) and triethylamine (2 × 0.23 ml) in dichloromethane as in previous examples. After purification, (2E,4E)-N-isobutyl 6-(1,2,3,4-tetrahydronaphtha-2-yl) hexa-2,4-dienamide was obtained as a colourless solid. m.p. 122°) Tlc: (Silica-ether), 1 spot, $R_f$ 0.43;

NMR: 7.03 (5H), M, aryl, H3; 6.10 (2H), m, H4, 5; 5.76 (1H), d, H2; 3.23 (2H), d of d; 0.94 (6H), d, isobtuyl; 2.74 (4H), m, benzylic; 2.17 (2H), m, allylic; 1.8 (4H), m, ring protons, $Bu^i$; 5.6H (1H), NH.

Example 79

(2E),(4E)-N-isobutyl 6(indan-2-yl)hexa-2,4-dienamide

3-Benzoylpropionic acid (35.6 g) was added to potassium carbonate (27.6 g) in water (100 ml). After solution, 37% aqueous formaldehyde (6 g) was added and the mixture stirred for 4 days. Conc. hydrochloric acid (50 ml) was added and the whole heated at 100° for 30 mins. After extraction into dichloromethane, washing with aqueous sodium carbonate and standard work-up, the crude product was purified by column chromatography (silica/ether) to yield 4-benzoyl-butyrolactone.

The above lactone (13 g) was heated for 9 mins at 100° with conc. sulphuric acid (70 ml). The mixture was poured onto ice and the solid product collected, dried and extracted with hot benzene. Filtration, cooling and removal of solvent from the filtrate yielded indanone acetic acid.

The above acid (7.1 g) in glacial acetic acid (90 ml) was hydrogenated in the presence of 10% palladium on carbon (1.4 g) to give indan-2-yl acetic acid.

The above acetic acid was subject to the same sequence of transformation as described in Example 69 to give ultimately (2E,4E)-N-isobutyl 6-indan-2-yl)hexa-2,4-dienamide as a colourless solid. m.p. 149°. Tlc; 1 spot, $R_f$0.48.

NMR; 7.05 (5H), m, aryl, H3; 6.05 (2H), m, H4, 5; 5.80 (1H), d, H2; 5.98 (1H); NH; 3.23 (2H), d of d; 1.8 (1H), m; 0.94 (6H), d, isobutyl; 2.76 (4H), m, benzylic; 2.36 (3H), m, allylic, H2[1].

Examples 78 to 89

The compounds of Examples 78 and 79, and the following compounds prepared using procedures analogous to those of Examples 78 and 79, are described in Table 2 below:

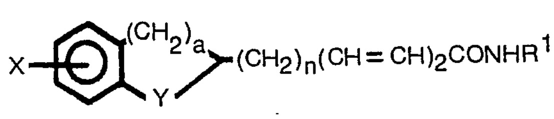

| Example No. | X | Y | a | $n^1$ | $R^1$ | Ppd as in example |
|---|---|---|---|---|---|---|
| 78 | H | $(CH_2)_2$ | 1 | 1 | isobutyl | — |
| 79 | H | $CH_2$ | 1 | 1 | isobutyl | — |
| 80 | H | $CH_2$ | 1 | 1 | 1,2-dimethylpropyl | 78 |
| 81 | H | $(CH_2)_2$ | 1 | 1 | 2-methyl-prop-2-enyl | 78 |
| 82 | H | $CH_2$ | 1 | 1 | 1,2-dimethylpropyl | 79 |
| 83 | 7—F | $(CH_2)_2$ | 1 | 1 | 1,2-dimethylpropyl | 78 |
| 84 | 7—Cl | $(CH_2)_2$ | 1 | 1 | isobutyl | 78 |
| 85 | 5,6- 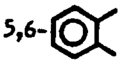 | $(CH_2)_2$ | 1 | 1 | isobutyl | 78 |
| 86 | 5,6- 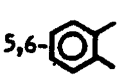 | $(CH_2)_2$ | 1 | 1 | 1,2-dimethylpropyl | 78 |
| 87 | H | $(CH_2)_3$ | 0 | 0 | isobutyl | |
| 88 | H | $(CH_2)_3$ | 0 | 0 | 1,2-dimethylpropyl | |
| 89 | H | $—OCH_2—$ | 0 | 4 | isobutyl | |

The following two compounds were also made by methods analogous to those described above: N,N-di([2-(1,3-dioxalano)]-methyl]) 6-phenyl-hexa-(2E,4E)-dienamide (Example 90) and N-methyl N-(1,2-dimethylpropyl) 6-phenylhexa-(2E,4E)-dienamide (Example 91).

Further nmr data

Example 8: mp. 136.5—137.5°; NMR: 7.22 (6H;, m, aryl, H3; 6.18 (2H), m, H4, 5; 5.78 (1H), d, H2; 5.60 (1H), NH; 3.44 (2H), d, H6; 3.96 (1H), m, 1.64 (1H), m, 1.12 (3H), d, 0.92 (6H), d, 1,2-dimethylpropyl

Example 14: NMR: 7.18 (6H), m, aryl, H3; 6.12 (2H), m, H4, 5; 5.82 (1H), d, H2; 5.90 (1H), NH; 2.59 (2H), t, H8; 2.16 (2H), m, H6; 1.85 (3H), m, H7, 1,2-dimethylpropyl; 3.98 (1H), m, 1.12 (3H), d, 0.92 (6H), 1,2-dimethylpropyl

Example 80: m.p. 123.5; NMR: 7.02 (5H), m, aryl, H3; 6.11 (2H), m, H4, 5; 5.78 (1H), d, H2; 5.64 (1H), NH; 2.75 (4H), m, benzylic; 2.20 (2H), m, H6; 1.8 (3H), m, ring hydrogens; 3.96 (1H), m, 1.63 (1H), 1.14 (3H), d, 0.94 (6H), d; 1,2-dimethylpropyl

## BIOLOGICAL EXAMPLES

A. Topical application to housefly (*Musca domestica*)

The compounds were administered topically to female *Musca domestica* in cellosolve solution, either alone or in conjunction with a synergist (6 μg piperonyl butoxide). The flies were kept with sugar water and the mortality was assessed after 24 hours. The results are given in Table A (first two columns):

B. Activity against grain pests

The compounds were applied (1:5 compound:piperonyl butoxide) in acetone to grain. When dry, the grain was infested with *Sitophilus granarius* or *Tribolium castaneum*. The insect mortality was assessed after 7 days to give an $LC_{50}$ figure in ppm. (Table A, columns 3 and 4).

C. Knockdown activity against insect

Solutions of the compounds were made up in OPD (odourless petroleum distillate)/dichloromethane and sprayed into a Kearns and March chamber, for *M. domestica*, or directly onto *Blattella germanica* or into a wind tunnel in which *Culex quinquefasciatus* were released. The time for knockdown of 50% of the insects was measured, and the concentration ($KC_{50}$) required for 50% knockdown in 4 minutes was calculated. The compound was used alone against *B. germanica*, but 1:5 compound:piperonyl butoxide against *M. domestica* and *C. quinquefasciatus*. The results are given in columns 5, 6 and 7 of Table A.

TABLE A

| Example No. | M domestica alone | +6µg PB | Pipette on $LC_{50}$ (ppm) S. gran | T. cast | $KC_{50}$ (4 mins) Musca | Blattella | Culex |
|---|---|---|---|---|---|---|---|
| 1 | <6 | <0.6 | <50 | c200 | <0.3 | | <0.1 |
| 2 | <6 | <2 | | | | | |
| 3 | | <0.5 | | | <0.3 | | <0.1 |
| 4 | | >20 | | | | | |
| 5 | <6 | <0.6 | | | <1 | <0.3 | <0.3 |
| 6 | <3 | <0.75 | <50 | c200 | | | |
| 7 | | <3 | <200 | | <1 | | |
| 8 | <6 | <0.2 | <20 | <200 | <0.5 | | <0.3 |
| 9 | <6 | <1 | | | <0.3 | <0.3 | <0.3 |
| 10 | <6 | <0.6 | <200 | c200 | <0.3 | <0.3 | <0.3 |
| 11 | | <10 | | | | | |
| 12 | <10 | <2 | <50 | | <0.3 | <0.3 | <0.3 |
| 13 | | <10 | <200 | | | <1 | |
| 14 | | <6 | <50 | | <1 | <0.3 | <0.3 |
| 15 | | >10 | | | | | |
| 16 | | <6 | | | | | |
| 17 | | <6 | | | | | |
| 18 | <6 | <1 | | | <1 | | <0.3 |
| 19 | <10 | <1 | | | <1 | <0.1 | <0.3 |
| 20 | | <5 | <50 | | | | |
| 21 | | >3 | | | | | |
| 22 | | <10 | | | | | |
| 23 | | <1 | <50 | | <1 | <0.3 | <0.3 |
| 24 | | <1 | <200 | | <1 | | <0.3 |
| 25 | | <3 | <50 | | | | |
| 26 | <20 | <6 | | | | | |
| 27 | | <3 | | | | | |
| 28 | | <3 | | | | | |
| 29 | | c20 | | | | | |

TABLE A (continued)

| Example No. | M domestica alone | +6µg PB | Pipette on $LC_{50}$ (ppm) S. gran | T. cast | $KC_{50}$ (4 mins) Musca | Blattella | Culex |
|---|---|---|---|---|---|---|---|
| 30 | | >20 | | | | | |
| 31 | | <6 | <200 | | | | <0.3 |
| 32 | c6 | <6 | <200 | | | | |
| 33 | | <3 | | | | | |
| 34 | | <2 | | | <1 | | <1 |
| 35 | | >6 | <200 | c200 | | | |
| 36 | | <3 | | | | | <0.3 |
| 37 | | >3 | | | <0.1 | | <0.3 |
| 38 | | <3 | | | | | |
| 39 | | <1 | | | | | |
| 40 | | <3 | <200 | | <0.1 | | <0.3 |
| 41 | | c3 | | | <0.1 | | |
| 42 | | <1 | <200 | c200 | | | <1 |
| 43 | | >3 | | | | | |
| 44 | | <5 | | | | | <1 |
| 45 | | <3 | | | | | <1 |
| 46 | | c3 | | | | | |
| 47 | | <3 | | | | | |
| 48 | | <3 | | | | | |
| 49 | | c3 | | | | | |
| 50 | | c3 | <200 | | | | |
| 51 | | <6 | | | | | |
| 52 | | <3 | <200 | | | | |
| 53 | | <3 | | | | | |
| 54 | | <1 | | | | | <1 |
| 55 | | <1 | <200 | | | | |
| 56 | | >3 | | | | | |
| 57 | | <3 | <200 | | | | |
| 58 | | c6 | | | | | |

TABLE A (continued)

| Example No. | M domestica alone | M domestica +6µg PB | Pipette on $LC_{50}$ (ppm) S. gran | T. cast | $KC_{50}$ (4 mins) Musca | Blattella | Culex |
|---|---|---|---|---|---|---|---|
| 59 | | >6 | | | | | |
| 60 | | >6 | <200 | | | | |
| 61 | | >3 | | | | | |
| 62 | | >6 | | | | | |
| 63 | | c6 | | | | | |
| 64 | | >5 | | | | | |
| 65 | | <10 | | | | | |
| 66 | | >1 | | | | | |
| 67 | | >5 | | | | | |
| 68 | | c5 | | | | | |
| 69 | | >5 | c200 | | | | |
| 70 | | <6 | | | | | |
| 71 | | <10 | | | | | |
| 72 | | <6 | | | | | |
| 73 | | <6 | | | | | |
| 74 | | <6 | | | | | |
| 75 | | >0.6 | | | | | |
| 76 | | >3 | | | | | |
| 77 | | <10 | | | | | |
| 78 | <6 | <1 | <50 | | <1 | | |
| 79 | <10 | <3 | | | <1 | | <1 |
| 80 | | <0.5 | | | <0.3 | | <0.3 |
| 81 | | <3 | <50 | | <1 | <0.1 | |
| 82 | | <0.6 | | | <1 | | <0.3 |
| 83 | <3 | <0.6 | | | | | |
| 84 | | <0.2 | <200 | | | | |
| 85 | | <3 | | | | | |
| 86 | | <0.2 | | | | | |

TABLE A (continued)

| Example No. | M domestica alone | M domestica +6µg PB | Pipette on $LC_{50}$ (ppm) S. gran | T. cast | $KC_{50}$ (4 mins) Musca | Blattella | Culex |
|---|---|---|---|---|---|---|---|
| 87 | | <3 | | | | | |
| 88 | | <6 | | | | | |
| 89 | | >3 | | | | | |

D. Acaricidal activity

The compounds were tested by injecting 10 µg in cellosolve into female *Boophilus microplus* adults and assessing the percentage inhibition of reproduction (%IR) over 2 weeks. The results are given in Table B:

TABLE B

| Compound of Example No. | % IR | Compound of Example No. | % IR |
|---|---|---|---|
| | | 42 | 90 |
| | | 43 | 0 |
| | | 44 | 40 |
| 2 | 20 | 45 | 100 |
| | | 46 | 45 |
| 6 | 70 | 47 | 100 |
| 7 | 0 | | |
| 8 | 100 | 49 | 60 |
| | | 50 | 0 |
| | | 52 | 100 |
| 15 | 20 | 53 | 100 |
| | | 54 | 100 |
| 16 | 10 | 55 | 100 |
| | | 56 | 50 |
| 18 | 100 | 57 | 20 |
| 19 | 0 | 58 | 90 |
| 20 | 50 | 59 | 30 |
| 21 | 70 | | |
| 23 | 0 | 66 | 10 |
| 24 | 100 | | |
| 25 | 10 | 68 | 0 |
| | | 69 | 10 |
| 27 | 0 | 70 | 10 |
| 28 | 10 | 71 | 90 |
| 31 | 0 | | |
| 32 | 60 | | |
| 33 | 50 | | |
| | | 78 | 70 |
| 34 | 10 | | |
| | | 86 | 80 |
| 37 | 0 | 82 | 100 |

Formulations

1. *Emulsifiable Concentrate*

| | |
|---|---|
| Compound of Example 1 | 10.00 |
| Ethylan KFO | 20.00 |
| Xylene | 67.50 |
| Butylated Hydroxyanisole | 2.50 |
| | 100.00 |

2. *Wettable Powder*

| | |
|---|---|
| Compound of Example 1 | 25.0 |
| Attapulgite | 69.50 |
| Sodium isopropylbenzene sulphonate | 0.50 |
| Sodium salt of condensed naphthalene sulphonic acid | 2.50 |
| Butylated hydroxytoluene | 2.50 |
| | 100.00 |

3. *Dust*

| | |
|---|---|
| Compound of Example 1 | 0.50 |
| Butylated Hydroxyanisole | 0.10 |
| Talc | 99.40 |
| | 100.00 |

4. *Bait*

| | |
|---|---|
| Compound of Example 1 | 40.25 |
| Icing Sugar | 99.65 |
| Butylated hydroxy toluene | 0.10 |
| | 100.00 |

5. *Lacquer*

| | |
|---|---|
| Compound of Example 1 | 2.5 |
| Resin | 5.0 |
| Butylated Hydroxy anisole | 0.5 |
| High aromatic white spirit | 92.0 |
| | 100.00 |

6. *Aerosol*

| | |
|---|---|
| Compound of Example 1 | 0.30 |
| Butylated Hydroxy anisole | 0.10 |
| 1,1,1-Trichloroethane | 4.00 |
| Odourless Kerosene | 15.60 |
| Arcton 11/12. 50:50 mix | 80.00 |
| | 100.00 |

7. *Spray*

| | |
|---|---|
| Compound of Example 1 | 0.1 |
| Butylated Hydroxy anisole | 0.1 |
| Xylene | 10.0 |
| Odourless Kerosene | 89.8 |
| | 100.00 |

8. *Potentiated Spray*

| | |
|---|---|
| Compound of Example 1 | 0.1 |
| Permethrin | 0.1 |
| Butylated Hydroxyanisole | 0.1 |
| Xylene | 10.1 |
| Odourless Kerosene | 89.8 |
| | 100.00 |

**Claims for the Contracting States: GB CH DF FR IT LI NL**

1. A compound of Formula (I):

$$Ar(CH_2)_n(CR^2{=}CR^3)_2\overset{\overset{O}{\|}}{C}NRR^1 \qquad (I)$$

wherein: Ar is phenyl, naphthyl, thienyl, fluorenyl, phenanthrenyl, dibenzo furanyl or a polynuclear group (A):

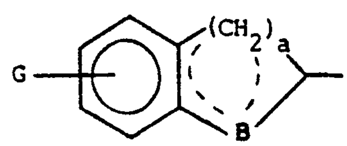

in which

a is 0, 1 or 2; B is $(D)_b(CH_2)_c(E)_e$ where each of D and E is oxygen or sulphur, b and e are independently 0 or 1 but not 1, and c is 0, 1, 2 or 3, the sum of a, b, c and e being at least 2, and the ring containing B is wholly or partially saturated; and G is hydrogen or a benzene ring fused to the benzene ring of group (A);

any of the groups Ar may be substituted by one or more of $C_{1-4}$ alkyl, halo-($C_{1-4}$) alkyl, halo, $C_{1-4}$ alkoxy (except 3,4-methylenedioxy) or $C_{1-4}$ halo -alkoxy; n is 1 to 8, except that n is 1 to 4 when Ar is phenyl or substituted phenyl;

each of $R^2$ and $R^3$ is in each case independently hydrogen, $C_{1-4}$ alkyl or halo($C_{1-4}$)alkyl; and R and $R^1$ are each selected from hydrogen, $C_{1-6}$ alkyl, $C_{3-6}$ cycloalkyl, $C_{2-6}$ alkenyl or $C_{1-6}$ alkoxy (any of which may

be substituted by halo, $C_{2-6}$ alkenyl, $C_{1-6}$ alkyl, $C_{3-6}$ cycloalkyl, $C_{1-6}$ alkoxy, $C_{2-6}$ alkynyl or cyano) except that the following compounds are excluded:

N-isobutyl-6-(2-naphthyl)-hexa-2E,4E-dienamide
N-(2,2-dimethylpropyl)-6-(2-naphthyl)-hexa-2E,4E-dienamide
N-isobutyl-6-(2-fluorenyl)-hexa-2E,4E-dienamide
N-(2,2-dimethyl-but-3-enyl)-6-(2-naphthyl)-hexa-2E,4E-dienamide
N-(2-methylbutyl)-6-(2-naphthyl)-hexa-2E,4E-dienamide
N-isobutyl-6-(2-phenanthrenyl)-hexa-2E,4E-dienamide
N-isobutyl-6-(5-bromo-2-naphthyl)-hexa-2E,4E-dienamide
N-isobutyl-6-(6-bromo-2-naphthyl)-hexa-2E,4E-dienamide
N-isobutyl-6-(2-dibenzofuranyl)-hexa-2E,4E-dienamide
N-(1,2,2-trimethylpropyl)-6-(2-napthyl)-hexa-2E,4E-dienamide
N-(1,2-dimethylpropyl)-6-(2-naphthyl)-hexa-2E,4E-dienamide
N-(2,2-dimethylpropyl)-6-(2-phenanthrenyl)-hexa-2E,4E-dienamide
N-(1,2-dimethylpropyl)-6-(2-phenanthrenyl)-hexa-2E,4E-dienamide
N-(2-methylbutyl)-6-(2-phenanthrenyl)-hexa-2E,4E-dienamide
N-(2,2-dimethylpropyl)-6-(5-bromo-2-naphthyl)-hexa-2E,4E-dienamide
N-(2-methylbutyl)-6-(5-bromo-2-naphthyl)-hexa-2E,4E-dienamide
N-(2,2-dimethylpropyl)-6-(2-dibenzofuranyl)-hexa-2E,4E-dienamide
N-(2,2-dimethylbut-3-enyl)-6-(2-dibenzofuranyl)-hexa-2E,4E-dienamide
N-(2,2-dimethylbut-3-enyl)-6-(2-phenanthrenyl)-hexa-2E,4E-dienamide
N-(1,2-dimethylpropyl)-6-(2-dibenzofuranyl)-hexa-2E,4E-dienamide
N-(2-methylbutyl)-6-(2-dibenzofuranyl)-hexa-2E,4E-dienamide
N-(1,2-dimethylpropyl)-6-(5-bromo-2-naphthyl)-hexa-2E,4E-dienamide
N-(2,2-dimethylbut-3-enyl)-6-(5-bromo-2-naphthyl)-hexa-2E,4E-dienamide
N-(2,2-dimethylpropyl)-6-(9-bromo-3-phenanthrenyl)-hexa-2E,4E-dienamide
N-(2,2-dimethylpropyl)-6-(5,8-dibromo-2-naphthyl)-hexa-2E,4E-dienamide
N-(2,2-dimethylpropyl)-6-(7-bromo-2-naphthyl)-hexa-2E,4E-dienamide
N-(2,2-dimethylpropyl)-6-(7-chloro-2-naphthyl)-hexa-2E,4E-dienamide
N-(2-methylpropyl)-6-(7-chloro-2-naphthyl)-hexa-2E,4E-dienamide
N-(2,2-dimethylpropyl)-6-(7-fluoro-2-naphthyl)-hexa-2E,4E-dienamide
N-(2,2-dimethylbut-3-enyl)-6-(9,10-dihydrophenanthrenyl)-hexa-2E,4E-dienamide
N-(1,2-dimethylpropyl)-6-(7-fluoro-2-naphthyl)-hexa-2E,4E-dienamide
N-(3,3-dimethylpropyl)-6-(6-chloro-2-naphthyl)-hexa-2E,4E-dienamide
N-isobutyl 7-phenylhepta-(2E,4E)-dienamide
N-isobutyl 6-phenylhexa-(2E,4E)-dienamide
N-isobutyl 6-(4-methoxyphenyl)hexa-(2E,4E)-dienamide
N-isobutyl 6-(2-thienyl)hexa-(2E,4E)-dienamide
N-isobutyl 8-phenylocta-(2E,4E)-dienamide
N-isobutyl 6-(1-naphthyl)hexa-(2E,4E)-dienamide

2. A compound of Formula (IA):

$$Ar—(CH_2)_n(CR^2{=}CR^3)_2C(O)NRR^1 \qquad (IA)$$

wherein Ar, n, R, $R^1$, $R^2$ and $R^3$ are defined above except that, (i) when n is 1 and R is H and Ar is 2-fluorenyl, 2-phenanthrenyl, 2-dibenzofuranyl, 9,10-dihydro-2-phenanthrenyl, 5-, 6- or 7-halo-2-naphthyl, 5,8-dibromo-2-naphthyl or 3-(9-bromo)-phenanthrenyl then $R^1$ is not isobutyl, 2,2-dimethylpropyl, 2,2-dimethyl-3-butenyl, 2-methylbutyl, 1,2,2-trimethylpropyl or 1,2-dimethylpropyl, and (ii) when n is 1 and R is H and Ar is 2-naphthyl then $R^1$ is not 2,2-dimethylpropyl, 2,2-dimethyl-3-butenyl, 2-methylbutyl or 1,2,2-trimethylpropyl.

3. A compound of Formula (IB):

$$Ar—(CH_2)_n(CR^2{=}CR^3)_2\overset{\overset{\displaystyle O}{\|}}{C}NRR^1 \qquad (IB)$$

wherein Ar, n, R, $R^1$, $R^2$, and $R^3$ are as defined above,
except that, when n is 1 and R is H and Ar is either a polycyclic wholly aromatic ring system joined at the 2-position or 3-phenanthrenyl, then $R^1$ is not isobutyl, 2,2-dimethylpropyl, 2,2-dimethyl-3-butenyl, 2-methylbutyl, 1,2,2-trimethylpropyl or 1,2-dimethylpropyl, with the proviso that N-isobutyl 8-(2-naphthyl)-octa-2E,4E-dienamide and N-(1,2-dimethylpropyl)-8-(2-naphthyl)-octa-2E,4E-dienamide are not excluded.

4. A compound according to any one of claims 1 to 3 wherein n is odd.

5. A compound according to any one of claims 1 to 4 wherein the configuration of both double bonds in the diene group is E.

6. A compound according to any one of claims 1 to 5 wherein Ar is phenyl, furyl, thienyl, benzofuranyl, benzopyranyl, chromanyl, indanyl, tetrahydronaphthyl, or any of the following groups:

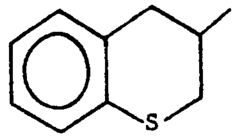
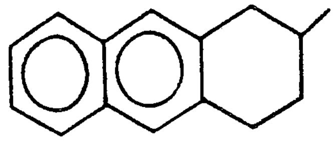

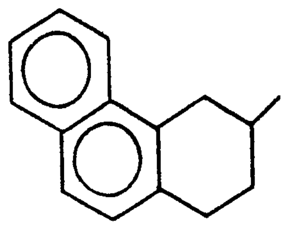
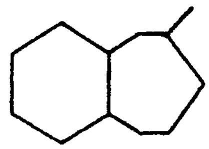

any of which may be substituted as in claims 1 to 5.

7. A compound according to claim 6, wherein Ar is phenyl or phenyl substituted at the 3-position by halo, haloalkyl or alkoxy or Ar is 3,4-dihalophenyl.

8. A compound according to any one of claims 1 to 5 wherein R is hydrogen and $R^1$ is isobutyl, 1-methylpropyl, 2,2-dimethylpropyl, 1,2,2-trimethylpropyl or 1,2-dimethylpropyl.

9. A compound according to any one of claims 1 to 6 wherein $R^2$ and $R^3$ are in each case hydrogen.

10. A process for preparing a compound according to any one of claims 1 to 9 by

(a) by reaction of a compound of Formula (II) with a compound of Formula (III):

$$Ar(CH_2)_n(CR^2{=}CR^3)_2\ COZ' \qquad (II)$$

$$HNRR^1 \qquad (III)$$

wherein $Z^1$ is hydroxyl, halo or a phosphoroimidate ester group

$$(\text{—}\overset{O^-}{\overset{|}{P}}(O\ Aryl)NH\ aryl)$$

and the other variables are as defined in claim 1;

(b) by reaction of a compound of Formula (IV) with a compound of Formula (V) or (VI);

$$Ar(CH_2)_n(CR^2{=}CR^3)_p\overset{O}{\overset{\|}{C}}H \qquad (IV)$$

$$(Z'')_3P{=}CH(CR^2{=}CHR^3)_qC(O)NRR^1 \qquad (VI)$$

$$(Z'')_2\underset{O^-}{\underset{|}{P}}{=}CH(CR^2{=}CR^3)_qC(O)NRR^1 \qquad (VI)$$

wherein Z'' is alkyl, alkoxy (preferably ethoxy) or aryl (preferably phenyl), and p + q = 1. The locations of the aldehyde and the phosphorus containing groups, $(Z'')_3P$ and $(Z'')_2P(O)$, may be swapped to give an exactly analogous reaction;

(c) β-elimination from a compound of Formula (VII) or (VIII):

$$Ar(CH_2)_n(CR^2{=}CR^3)\overset{X}{\overset{|}{C}}R^2\overset{Y}{\overset{|}{C}}R^3C(O)NRR^1 \qquad (VII)$$

$$Ar(CH_2)_n\overset{X}{\overset{|}{C}}R^2\overset{Y}{\overset{|}{C}}R^3(CR^2{=}CR^3)C(O)NRR^1 \qquad (VIII)$$

wherein one of X and Y is hydrogen and the other is a group $Q(+O^-)L$, where Q is sulphur or selenium and L is a suitable group;

(d) reaction of a compound of Formula (IX) with a compound of Formula (X):—

$$Ar(CH_2)_nCH_2\text{-}Hal \qquad (IX)$$

$$R^2C{\equiv}CR^3(CR^2{=}CR^3)C(O)NR^1 \qquad (X)$$

where Hal is a halogen atom, followed by reduction of the triple bond; or

(e) by reacting a compound of Formula (XI) with a compound of Formula (XII):

$$Ar(CH_2)_nCR^2{=}CR^3\text{—}M \qquad (XI)$$

$$Hal\text{-}(CR^2{=}CR^3)\overset{\overset{O}{\|}}{C}NRR^1 \qquad (XII)$$

wherein Hal is halide and M is a metal atom or metal group,

11. A pesticidal composition comprising a compound

$$Ar\text{—}(CH_2)_n(CR^2{=}CR^3)_2\overset{\overset{O}{\|}}{C}NRR^1 \qquad (I)$$

wherein: Ar is phenyl, naphthyl, thienyl, fluorenyl, phenanthrenyl, dibenzofuranyl or a polynuclear group (A):

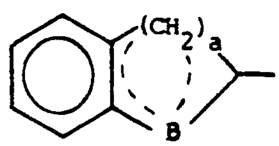

in which

a is 0, 1 or 2; B is $(D)_b(CH_2)_c(E)_e$ where ech of D and E is oxygen or sulphur, b and e are independently 0 or 1 but not 1, and c is 0, 1, 2 or 3, the sum of a, b, c and e being at least 2, and the ring containing B is wholly or partially saturated; and G is hydrogen or a benzene ring fused to the benzene ring of group (A);

any of the groups Ar may be substituted by one or more of $C_{1-4}$ alkyl, halo-($C_{1-4}$) alkyl, halo, $C_{1-4}$ alkoxy (except 3,4-methylenedioxy) or $C_{1-4}$ halo -alkoxy; n is 1 to 8, except that n is 1 to 4 when Ar is phenyl or substituted phenyl;

each of $R^2$ and $R^3$ is in each case independently hydrogen, $C_{1-4}$ alkyl or halo($C_{1-4}$)alkyl; and R and $R^1$ are each selected from hydrogen, alkyl, cycloalkyl, alkenyl or alkoxy (any of which may be substituted by halo, alkenyl, alkyl, cycloalkyl, alkoxy, alkynyl or cyano) except that the following compounds are excluded.

N-isobutyl-6-(2-naphthyl)-hexa-2E,4E-dienamide
N-(2,2-dimethylpropyl)-6-(2-naphthyl)-hexa-2E,4E-dienamide
N-isobutyl-6-(2-fluorenyl)-hexa-2E,4E-dienamide
N-(2,2-dimethyl-but-3-enyl)-6-(2-naphthyl)-hexa-2E,4E-dienamide
N-(2-methylbutyl)-6-(2-naphthyl)-hexa-2E,4E-dienamide
N-isobutyl-6-(2-phenanthrenyl)-hexa-2E,4E-dienamide
N-isobutyl-6-(5-bromo-2-naphthyl)-hexa-2E,4E-dienamide
N-isobutyl-6-(6-bromo-2-naphthyl)-hexa-2E,4E-dienamide
N-isobutyl-6-(2-dibenzofuranyl)-hexa-2E,4E-dienamide
N-(1,2,2-trimethylpropyl)-6-(2-napthyl)-hexa-2E,4E-dienamide
N-(1,2-dimethylpropyl)-6-(2-naphthyl)-hexa-2E,4E-dienamide
N-(2,2-dimethylpropyl)-6-(2-phenanthrenyl)-hexa-2E,4E-dienamide
N-(1,2-dimethylpropyl-6-(2-phenanthrenyl)-hexa-2E,4E-dienamide
N-(2-methylbutyl)-6-(2-phenanthrenyl)-hexa-2E,4E-dienamide
N-(2,2-dimethylpropyl)-6-(5-bromo-2-naphthyl)-hexa-2E,4E-dienamide
N-(2-methylbutyl)-6-(5-bromo-2-naphthyl)-hexa-2E,4E-dienamide
N-(2,2-dimethylpropyl)-6-(2-dibenzofuranyl)-hexa-2E,4E-dienamide
N-(2,2-dimethylbut-3-enyl)-6-(2-dibenzofuranyl)-hexa-2E,4E-dienamide
N-(2,2-dimethylbut-3-enyl)-6-(2-phenanthrenyl)-hexa-2E,4E-dienamide
N-(1,2-dimethylpropyl)-6-(2-dibenzofuranyl)-hexa-2E,4E-dienamide
N-(2-methylbutyl)-6-(2-dibenzofuranyl)-hexa-2E,4E-dienamide
N-(1,2-dimethylpropyl-6-(5-bromo-2-naphthyl)-hexa-2E,4E-dienamide
N-(2,2-dimethylbut-3-enyl)-6-(5-bromo-2-naphthyl)-hexa-2E,4E-dienamide
N-(2,2-dimethylpropyl)-6-(9-bromo-3-phenanthrenyl)-hexa-2E,4E-dienamide
N-(2,2-dimethylpropyl)-6-(5,8-dibromo-2-naphthyl)-hexa-2E,4E-dienamide
N-(2,2-dimethylpropyl)-6-(7-bromo-2-naphthyl)-hexa-2E,4E-dienamide
N-(2,2-dimethylpropyl)-6-(7-choro-2-naphthyl)-hexa-2E,4E-dienamide

N-(2-methylpropyl)-6-(7-chloro-2-naphthyl)-hexa-2E,4E-dienamide
N-(2,2-dimethylpropyl)-6-(7-fluoro-2-naphthyl)-hexa-2E,4E-dienamide
N-(2,2-dimethylbut-3-enyl)-6-(9,10-dihydrophenanthrenyl)-hexa-2E,4E-dienamide
N-(1,2-dimethylpropyl)-6-(7-fluoro-2-naphthyl)-hexa-2E,4E-dienamide
N-isobutyl 8-phenyl octa-(2E,4E)-dienamide
N-isobutyl 6-phenyl hexa-(2E,4E)-dienamide
N-isobutyl 6-(2-thienyl) hexa-(2E,4E)-dienamide

and one or more carriers.

12. A method of combatting pests by applying to a locus a compound according to any one of claims 1 to 9 or a composition according to claim 11.

13. N-(1,2-dimethylpropyl)-6-(indan-2-yl)hexa-2,4-dienamide.

**Claims for the Contracting State: AT**

1. A process for preparing a compound of Formula (I):

$$Ar(CH_2)_n(CR^2{=}CR^3)_2NRR^1 \quad (I)$$

wherein: Ar is phenyl, naphthyl, thienyl, fluorenyl, phenanthrenyl, dibenzofuranyl or a polynuclear group (A):

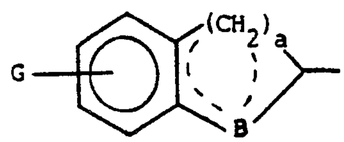

in which

a is 0, 1 or 2; B is $(D)_b(CH_2)_c(E)_e$ where each of D and E is oxygen or sulphur, b and e are independently 0 or 1 but not both 1, and c is 0, 1, 2 or 3, the sum of a, b, c and e being at least 2, and the ring containing B is wholly or partially saturated; and G is hydrogen or a benzene ring fused to the benzene ring of group (A);

any of the groups Ar may be substituted by one or more of $C_{1-4}$-alkyl, halo-$(C_{1-4})$-alkyl, halo, $C_{1-4}$-alkoxy (except 3,4-methylenedioxy) or $C_{1-4}$-halo-alkoxy; n is 1 to 8, except that n is 1 to 4 when Ar is phenyl or substituted phenyl;

each of $R^2$ and $R^3$ is in each case independently hydrogen, $C_{1-4}$-alkyl or halo-$(C_{1-4})$-alkyl; and R and $R^1$ are each selected from hydrogen, $C_{1-6}$-alkyl, $C_{3-6}$-cycloalkyl, $C_{2-6}$-alkenyl or $C_{1-6}$-alkoxy (any of which may be substituted by halo, $C_{2-6}$-alkenyl, $C_{1-6}$-alkyl, $C_{3-6}$-cycloalkyl, $C_{1-6}$-alkoxy, $C_{2-6}$-alkynyl or cyano) except for the following compounds:

N-isobutyl 7-phenylhepta-(2E,4E)-dienamide
N-isobutyl 6-phenylhexa-(2E,4E)-dienamide
N-isobutyl 6-(4-methoxyphenyl)hexa-(2E,4E)-dienamide
N-isobutyl 6-(2-thienyl)hexa-(2E,4E)-dienamide
N-isobutyl 8-phenylocta-(2E,4E)-dienamide
N-isobutyl 6-(1-naphthyl)hexa-(2E,4E)-dienamide

by:

(a) reaction of a compound of Formula (II) with a compound of Formula (III):

$$Ar(CH_2)_n(CR^2{=}CR^3)_2\,COZ' \quad (II)$$

$$HNRR^1 \quad (III)$$

wherein $Z^1$ is hydroxyl, halo or a phosphoroimidate ester group (—P ($O^-$) (O Aryl) NH aryl) and the other variables are as defined above;

(b) by reaction of a compound of Formula (IV) with a compound of Formula (V) or (VI);

$$Ar(CH_2)_n(CR^2{=}CR^3)_pC(O)H \quad (IV)$$

$$(Z'')_3P{=}CH(CR^2{=}CHR^3)_qC(O)NRR^1 \quad (V)$$

$$(Z'')_2\underset{\substack{|\\O^-}}{P}{=}CH(CR^2{=}CR^3)_qC(O)NRR^1 \quad (VI)$$

wherein Z'' is alkyl, alkoxy (preferably ethoxy) or aryl (preferably phenyl), and p + q = 1. The locations of the aldehyde and the phosphorus containing groups, $(Z'')_3P$ and $(Z'')_2P(O)$, may be swapped to give an exactly analogous reaction;

(c) β-elimination from a compound of Formula (VII) or (VIII):

$$Ar(CH_2)_n(CR^2{=}CR^3)\underset{}{C}R^2(X)CR^3(Y)C(O)NRR^1 \quad \text{(VII)}$$

$$Ar(CH_2)_nCR^2(X)CR^3(Y)(CR^2{=}CR^3)C(O)NRR^1 \quad \text{(VIII)}$$

wherein one of X and Y is hydrogen and the other is a group $Q(+O^-)L$, where Q is sulphur or selenium and L is a suitable group;

(d) reaction of a compound of Formula (IX) with a compound of Formula (X):—

$$Ar(CH_2)_nCH_2\text{-}Hal \quad \text{(IX)}$$

$$R^2C{\equiv}CR^3(CR^2{=}CR^3)C(O)NR^1 \quad \text{(X)}$$

where Hal is a halogen atom, followed by reduction of the triple bond; or

(e) by reacting a compound of Formula (XI) with a compound of Formula (XII):

$$Ar(CH_2)_nCR^2{=}CR^3\text{—}M \quad \text{(XI)}$$

$$Hal\text{-}(CR^2{=}CR^3)C({=}O)NRR^1 \quad \text{(XII)}$$

wherein Hal is halide and M is a metal atom or metal group,

2. A process according to claim 1 to wherein n is odd.

3. A process according to claim 1 or 2 wherein the configuration of both double bonds in the diene group is *E*.

4. A process according to any one of claims 1 to 3 wherein Ar is phenyl, furyl, thienyl, naphthyl, benzofuranyl, benzopyranyl, chromanyl, indanyl, tetrahydronaphthyl, or any of the following groups:

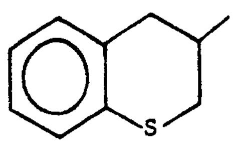

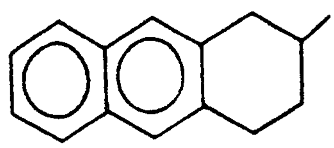

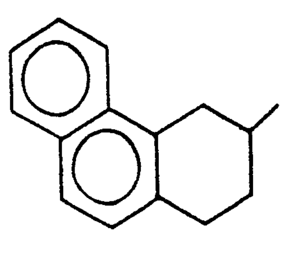

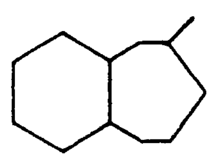

any of which may be substituted as in claims 1 to 3.

5. A process according to claim 4 wherein Ar is phenyl or phenyl substituted at the 3-position by halo, haloalkyl or alkoxy or Ar is 3,4-dihalophenyl.

6. A process according to any one of claims 1 to 5 wherein R is hydrogen and $R^1$ is isobutyl, 1-methylpropyl, 2,2-dimethylpropyl, 1,2,2-trimethylpropyl or 1,2-dimethylpropyl.

7. A process according to any one of claims 1 to 6 wherein $R^2$ and $R^3$ are in each case hydrogen.

8. A pesticidal composition comprising a compound prepared according to any one of claims 1 to 7 and one or more carriers.

9. A method of combatting pests by applying to a locus a compound prepared according to any one of claims 1 to 7 or a composition according to claim 8.

10. N-(1,2-dimethylpropyl)-6-(indan-2-yl)hexa-2,4-dienamide.

**Claims for the Contracting States: SE LU**

1. A compound of Formula (I):

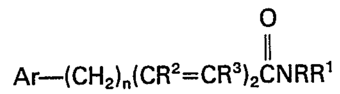

(I)

wherein: Ar is phenyl, naphthyl, thienyl, fluorenyl, phenanthrenyl, dibenzofuranyl or a polynuclear group (A):

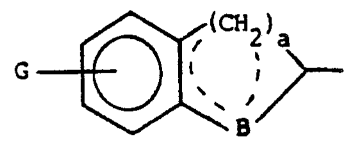

in which

a is 0, 1 or 2; B is $(D)_b(CH_2)_c(E)_e$ where ech of D and E is oxygen or sulphur, b and e are independently 0 or 1 but not both 1, and c is 0, 1, 2 or 3, the sum of a, b, c and e being at least 2, and the ring containing B is wholly or partially saturated; and G is hydrogen or a benzene ring fused to the benzene ring of group (A);

any of the groups Ar may be substituted by one or more of $C_{1-4}$ alkyl, halo-($C_{1-4}$) alkyl, halo, $C_{1-4}$ alkoxy (except 3,4-methylenedioxy) or $C_{1-4}$ halo -alkoxy; n is 1 to 8, except that n is 1 to 4 when Ar is phenyl or substituted phenyl;

each of $R^2$ and $R^3$ is in each case independently hydrogen, $C_{1-4}$ alkyl or halo-($C_{1-4}$)alkyl; and R and $R^1$ are each selected from hydrogen, $C_{1-6}$ alkyl, $C_{3-6}$ cycloalkyl, $C_{2-6}$ alkenyl or $C_{1-6}$ alkoxy (any of which may be substituted by halo, $C_{2-6}$ alkenyl, $C_{1-6}$ alkyl, $C_{3-6}$ cycloalkyl, $C_{1-6}$ alkoxy, $C_{2-6}$ alkynyl or cyano) except for the following compounds:

N-isobutyl 7-phenylhepta-(2E,4E)-dienamide
N-isobutyl 6-phenylhexa-(2E,4E)-dienamide
N-isobutyl 6-(4-methoxyphenyl)hexa-(2E,4E)-dienamide
N-isobutyl 6-(2-thienyl)hexa-(2E,4E)-dienamide
N-isobutyl 8-phenylocta-(2E,4E)-dienamide
N-isobutyl 6-(1-naphthyl)hexa-(2E,4E)-dienamide

2. A compound according to claim 1 wherein n is odd.

3. A compound according to claim 1 wherein the configuration of both double bonds in the diene group is *E*.

4. A compound according to any one of claims 1 to 3 wherein Ar is phenyl, furyl, thienyl, naphthyl, benzofuranyl, benzopyranyl, chromanyl, indanyl, tetrahydronaphthyl, or any of the following groups:

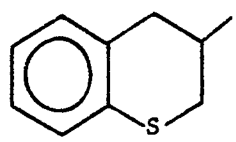

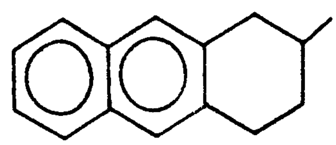

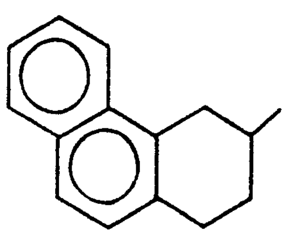

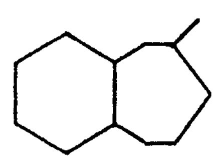

any of which may be substituted as in claims 1 to 3.

5. A compound according to claim 4 wherein Ar is phenyl or phenyl substituted at the 3-position by halo, haloalkyl or alkoxy or Ar is 3,4-dihalophenyl.

6. A compound according to any one of claims 1 to 5 wherein R is hydrogen and $R^1$ is isobutyl, 1-methylpropyl, 2,2-dimethylpropyl, 1,2,2-trimethylpropyl or 1,2-dimethylpropyl.

7. A compound according to any one of claims 1 to 6 wherein $R^2$ and $R^3$ are in each case hydrogen.

8. A process for preparing a compound according to any one of claims 1 to 7 by

(a) by reaction of a compound of Formula (II) with a compound of Formula (III):

$$Ar(CH_2)_n(CR^2{=}CR^3)_2\ COZ' \qquad (II)$$

$$HNRR^1 \qquad (III)$$

wherein $Z^1$ is hydroxyl, halo or a phosphoroimidate ester group

$$(-\overset{O^-}{\overset{|}{P}}(O\ Aryl)NH\ aryl)$$

and the other variables are as defined in claim 1;

(b) by reaction of a compound of Formula (IV) with a compound of Formula (V) or (VI);

$$Ar(CH_2)_n(CR^2{=}CR^3)_p\overset{O}{\overset{\|}{C}}H \qquad (IV)$$

$$(Z'')_3P{=}CH(CR^2{=}CHR^3)_qC(O)NRR^1 \qquad (V)$$

$$(Z'')_2\underset{O^-}{\underset{|}{P}}{=}CH(CR^2{=}CR^3)_qC(O)NRR^1 \qquad (VI)$$

wherein Z'' is alkyl, alkoxy (preferably ethoxy) or aryl (preferably phenyl), and $p + q = 1$. The locations of the aldehyde and the phosphorus containing groups, $(Z'')_3P$ and $(Z'')_2P(O)$, may be swapped to give an exactly analogous reaction;

(c) β-elimination from a compound of Formula (VII) or (VIII):

$$Ar(CH_2)_n(CR^2{=}CR^3)\overset{X}{\overset{|}{C}}R^2\overset{Y}{\overset{|}{C}}R^3C(O)NRR^1 \qquad (VII)$$

$$Ar(CH_2)_n\overset{X}{\overset{|}{C}}R^2\overset{Y}{\overset{|}{C}}R^3(CR^2{=}CR^3)C(O)NRR^1 \qquad (VIII)$$

wherein one of X and Y is hydrogen and the other is a group $Q(+O^-)L$, where Q is sulphur or selenium and L is a suitable group;

(d) reaction of a compound of Formula (IX) with a compound of Formula (X):—

$$Ar(CH_2)_nCH_2\text{-}Hal \qquad (IX)$$

$$R^2C{\equiv}CR^3(CR^2{=}CR^3)C(O)NR^1 \qquad (X)$$

where Hal is a halogen atom, followed by reduction of the triple bond; or

(e) by reacting a compound of Formula (XI) with a compound of Formula (XII):

$$Ar(CH_2)_nCR^2{=}CR^3{-}M \qquad (XI)$$

$$Hal\text{-}(CR^2{=}CR^3)\overset{O}{\overset{\|}{C}}NRR^1 \qquad (XII)$$

wherein Hal is halide and M is a metal atom or metal group,

9. A pesticidal composition comprising a compound

$$Ar{-}(CH_2)_n(CR^2{=}CR^3)_2\overset{O}{\overset{\|}{C}}NRR^1 \qquad (I)$$

wherein: Ar is phenyl, naphthyl, thienyl, fluorenyl, phenanthrenyl, dibenzofuranyl or a polynuclear group (A):

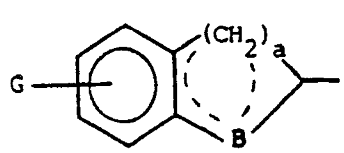

in which

a is 0, 1 or 2; B is $(D)_b(CH_2)_c(E)_e$ where each of D and E is oxygen or sulphur, b and e are independently 0 or 1 but not 1, and c is 0, 1, 2 or 3, the sum of a, b, c and e being at least 2, and the ring containing B is wholly or partially saturated; and G is hydrogen or a benzene ring fused to the benzene ring of group (A);

any of the groups Ar may be substituted by one or more of $C_{1-4}$ alkyl, halo-$(C_{1-4})$ alkyl, halo, $C_{1-4}$ alkoxy (except 3,4-methylenedioxy) or $C_{1-4}$ halo -alkoxy; n is 1 to 8, except that n is 1 to 4 when Ar is phenyl or substituted phenyl;

each of $R^2$ and $R^3$ is in each case independently hydrogen, $C_{1-4}$ alkyl or halo$(C_{1-4})$alkyl; and R and $R^1$ are each selected from hydrogen, $C_{1-6}$ alkyl, $C_{3-6}$ cycloalkyl, $C_{2-6}$ alkenyl or $C_{1-6}$ alkoxy (any of which may be substituted by halo, $C_{1-6}$ alkenyl, $C_{1-6}$ alkyl, $C_{3-6}$ cycloalkyl, $C_{1-6}$ alkoxy, $C_{2-6}$ alkynyl or cyano) and one or more carriers.

10. A method of combatting pests by applying to a locus a compound according to any one of claims 1 to 8 or a composition according to claim 9.

11. N-(1,2-dimethylpropyl)-6-(indan-2-yl)hexa-2,4-dienamide.

**Patentansprüche für die Vertragsstaaten: GB CH DE FR IT LI NL BE**

1. Verbindung der Formel (I):

$$Ar(CH_2)_n(CR^2{=}CR^3)_2\overset{\displaystyle O}{\overset{\|}{C}}NRR^1 \qquad (I)$$

worin: Ar Phenyl, Naphthyl, Thienyl, Fluorenyl, Phenanthrenyl, Dibenzofuranyl oder ein polykernige Gruppe (A) bedeutet:

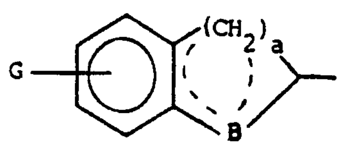

worin a 0, 1 oder 2 ist; B $(D)_b(CH_2)_c(E)_e$ ist, worin jeder Rest D und E Sauerstoff oder Schwefel ist, b und e unabhängig 0 oder 1 sind, aber nicht beide 1 sind, und c 0, 1, 2 oder 3 ist, wobei die Summe von a, b, c und e mindestens 2 ist, und der Ring, der B enthält, vollstandig oder teilweise gesättigt ist; und G Wasserstoff oder einen Benzolring beduetet, der an den Benzolring der Gruppe (A) kondensiert ist;

jeder Rest der Gruppe Ar kann durch eine oder mehrere von $C_{1-4}$-Alkyl, Halo-$C_{1-4}$)-alkyl, Halo, $C_{1-4}$-Alkoxy (ausgenommen 3,4-Methylendioxy) oder $C_{1-4}$-Halo-alkoxy substituiert sein; n 1 bis 8 ist, ausgenommen daß n 1 bis 4 ist, wenn Ar Phenyl oder substituiertes Phenyl bedeutet;

jeder Rest von $R^2$ und $R^3$ in jedem Fall unabhängig Wasserstoff, $C_{1-4}$-Alkyl oder Halo-$C_{1-4}$-alkyl bedeutet; und R und $R^1$ jeweils aus Wasserstoff, $C_{1-6}$-Alkyl, $C_{3-6}$-Cycloalkyl, $C_{2-6}$-Alkenyl oder $C_{1-6}$-Alkoxy ausgewählt sind (jeder Rest kann durch Halo, $C_{2-6}$-Alkenyl, $C_{1-6}$-Alkyl, $C_{3-6}$Cycloalkyl, $C_{1-6}$-Alkoxy, $C_{2-6}$-Alkinyl oder -Cyano substituiert sein), mit der Ausnahme, daß die folgenden Verbindungen ausgeschlossen sind:

N-Isobutyl-6-(2-naphthyl)-hexa-2F,4E-dienamid
N-(2,2-Dimethylpropyl)-6-(2-naphthyl)-hexa-2E,4E-dienamid
N-Isobutyl-6-(2-fluorenyl)-hexa-2E,4E-dienamid
N-(2,2-Dimethyl-but-3-enyl)-6-(2-naphthyl)-hexa-2E,4E-dienamid
N-(2-Methylbutyl)-6-(2-naphthyl)-hexa-2E,4E-dienamid
N-Isobutyl-6-(2-phenanthrenyl)-hexa-2E,4E-dienamid
N-Isobutyl-6-(5-brom-2-naphthyl)-hexa-2E,4E-dienamid
N-Isobutyl-6-(6-brom-2-naphthyl)-hexa-2E,4E-dienamid
N-Isobutyl-6-(2-dibenzofuranyl)-hexa-2E,4E-dienamid
N-(1,2,2,-Trimethylpropyl)-6-(2-naphthyl)-hexa-2E,4E-dienamid
N-(1,2-Dimethylpropyl)-6-(2-naphthyl)-hexa-2E,4E-dienamid
N-(2,2-Dimethylpropyl-6-(2-phenanthrenyl)-hexa-2E,4E-dienamid
N-(1,2-Dimethylpropyl)-6-(2-phenanthrenyl)-hexa-2E,4E-dienamid
N-(2-Methylbutyl)-6-(2-phenanthrenyl)-hexa-2E,4E-dienamid
N-(2,2-Dimethylpropyl)-6-(5-brom-2-naphthyl)-hexa-2E,4E-dienamid
N-(2-Methylbutyl)-6-(5-brom-2-naphthyl)-hexa-2E,4E-dienamid
N-(2,2-Dimethylpropyl)-6-(2-dibenzofuranyl-hexa-2E,4E-dienamid
N-(2,2-Dimethylbut-3-enyl)-6-(2-dibenzofuranyl)-hexa-2E,4E-dienamid
N-(2,2-Dimethylbut-3-enyl)-6-(2-phenanthrenyl)-hexa-2E,4E-dienamid
N-(1,2-Dimethylpropyl)-6-(2-dibenzofuranyl)-hexa-2E,4E-dienamid
N-(2-Methylbutyl)-6-(2-dibenzofuranyl)-hexa-2E,4E-dienamid
N-(1,2-Dimethylpropyl-6-(5-brom-2-naphthyl)-hexa-2E,4E-dienamid
N-(2,2-Dimethylbut-3-enyl)-6-(5-brom-2-naphthyl)-hexa-2E,4E-dienamid

N-(2,2-Dimethylpropyl)-6-(9-brom-3-phenanthrenyl)-hexa-2E,4E-dienamid
N-(2,2-Dimethylpropyl)-6-(5,8-dibrom-2-naphthyl)-hexa-2E,4E-dienamid
N-(2,2-Dimethylpropyl)-6-(7-brom-2-naphthyl)-hexa-2E,4E-dienamid
N-(2,2-Dimethylpropyl)-6-(7-chlor-2-naphthyl)-hexa-2E,4E-dienamid
N-(2-Methylpropyl)-6-(7-chlor-2-naphthyl)-hexa-2E,4E-dienamid
N-(2,2-Dimethylpropyl)-6-(7-fluor-2-naphthyl)-hexa-2E,4E-dienamid
N-(2,2-Dimethylbut-3-enyl)-6-(9,10-dihydrophenanthrenyl)-hexa-2E,4E-dienamid
N-(1,2-Dimethylpropyl)-6-(7-fluor-2-naphthyl)-hexa-2E,4E-dienamid
N-(3,3-Dimethylpropyl)-6-(6-chlor-2-naphthyl)-hexa-2E,4E-dienamid
N-Isobutyl-7-phenylhepta-(2E,4E)-dienamid
N-Isobutyl-6-phenylhexa-(2E,4E)-dienamid
N-Isobutyl-6-(4-methoxyphenyl)-hexa-(2E,4E)-dienamid
N-Isobutyl-6-(2-thienyl)-hexa-(2E,4E)-dienamid
N-Isobutyl-8-phenylocta-(2E,4E)-dienamid
N-Isobutyl-6-(1-naphthyl)-hexa-(2E,4E)-dienamid.

2. Verbindung der Formel (IA):

$$Ar\text{-}(CH_2)_n(CR^2{=}CR^3)_2\ C(O)NRR^1 \qquad (IA)$$

worin Ar, n, R, $R^1$, $R^2$ und $R^3$ die vorstehende Bedeutung Aufweisen, mit der Ausnahme, daß, (i) wenn n 1 ist und R H ist und Ar 2-Fluorenyl, 2-Phenanthrenyl, 2-Dibenzofuranyl, 9,10-Dihydro-2-phenanthrenyl, 5-, 6- oder 7-Halo-2-naphthyl, 5,8-Dibrom-2-naphthyl oder 3-(9-Brom)-phenanthrenyl ist, dann ist $R^1$ nicht Isobutyl, 2,2-Dimethylpropyl, 2,2-Dimethyl-3-butenyl, 2-Methylbutyl, 1,2,2-Trimethylpropyl oder 1,2-Dimethylpropyl, und (ii) wenn n 1 ist und R H ist und Ar 2-Naphthyl ist, dann ist $R^1$ nicht 2,2-Dimethylpropyl, 2,2-Dimethyl-3-butenyl, 2-Methylbutyl oder 1,2,2-Trimethylpropyl.

3. Verbindung der Formel (IB):

$$Ar\text{—}(CH_2)_n(CR^2{=}CR^3)_2\overset{\displaystyle O}{\overset{\|}{C}}NRR^1 \qquad (IB)$$

worin Ar, n, R, $R^1$, $R^2$ und $R^3$ die vorstehende Bedeutung besitzen, mit der Ausnahme, daß, wenn n 1 ist und R H ist und Ar entweder ein polycyclisches vollständig aromatisches Ringsystem, gebunden an die 2-Position oder 3-Phenanthrenyl ist, dann ist $R^1$ nicht Isobutyl, 2,2-Dimethylpropyl, 2,2-Dimethyl-3-butenyl, 2-Methylbutyl, 1,2,2-Trimethylpropyl oder 1,2-Dimethylpropyl mit der Maßgabe, daß N-Isobutyl-8-(2-naphthyl)-octa-2E,4E-dienamid und N-(1,2-Dimethylpropyl)-8-(2-naphthyl)-octa-2E,4E-dienamid nicht ausgeschlossen sind.

4. Verbindung nach einem der Ansprüche 1 bis 3, worin n eine ungerade Zahl ist.

5. Verbindung nach einem der Ansprüche 1 bis 4, worin die Konfiguration von beiden Doppelbindungen in der Diengruppe E ist.

6. Verbindung nach einem der Ansprüche 1 bis 5, worin Ar Phenyl, Furyl, Thienyl, Benzofuranyl, Benzopyranyl, Chromanyl, Indanyl, Tetrahydronaphthyl oder eine der folgenden Gruppen bedeutet:

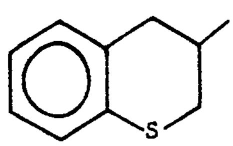

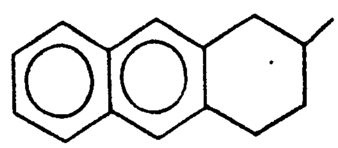

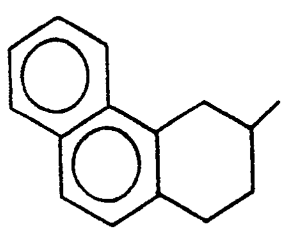

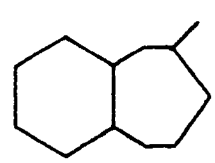

wobei jede wie in den Ansprüchen 1 bis 5 angegeben substituiert sein kann.

7. Verbindung nach Anspruch 6, worin Ar Phenyl oder Phenyl substituiert in der 3-Stellung durch Halo, Haloalkyl oder Alkoxy ist, oder Ar 3,4-Dihalophenyl bedeutet.

8. Verbindung nach einem der Ansprüche 1 bis 5 worin R Wasserstoff bedeutet und $R^1$ Isobutyl, 1-Methylpropyl, 2,2-Dimethylpropyl, 1,2,2-Trimethylpropyl oder 1,2-Dimethylpropyl bedeutet.

9. Verbindung nach einem der Ansprüche 1 bis 6, worin $R^2$ und $R^3$ in jedem Fall Wasserstoff sind.

10. Verfahren zur Herstellung einer Verbindung nach einem der Ansprüche 1 bis 9, durch

(a) Reaktion einer Verbindung der Formel (II) mit einer Verbindung der Formel (III):

$Ar(CH_2)_n(CR^2{=}CR^3)_2\ COZ'$ (II)

$HNRR^1$ (III)

worin $Z^1$ Hydroxyl, Halogen oder eine Phosphorimidatestergruppe

$O^-$
|
(—P(O Aryl) NH Aryl)

bedeutet und die anderen Variablen die in Anspruch 1 gegebene Bedeutung aufweisen;

(b) Reaktion einer Verbindung der Formel (IV) mit einer Verbindung der Formel (V) oder (VI):

O
‖
$Ar(CH_2)_n(CR^2{=}CR^3)_pCH$ (IV)

$(Z'')_3P{=}CH(CR^2{=}CHR^3)_qC(O)NRR^1$ (V)

$(Z'')_2P{=}CH(CR^2{=}CR^3)_qC(O)NRR^1$
|
$O^-$ (VI)

worin Z'' Alkyl, Alkoxy (vorzugsweise Ethoxy) oder Aryl (vorzugsweise Phenyl) bedeutet, und $p + q = 1$. Die Stellungen der aldehyd- und phosphorhaltigen Gruppen, $(Z'')_3O$ und $(Z'')_2P(O)$, können ausgetauscht werden, um eine exakt analoge Reaktion zu ergeben;

(c) β-Elimination aus einer Verbindung der Formel (VII) oder (VIII):

X Y
| |
$Ar(CH_2)_n(CR^2{=}CR^3)CR^2CR^3C(O)NRR^1$ (VII)

X Y
| |
$Ar(CH_2)_nCR^2CR^3(CR^2{=}CR^3)C(O)NRR^1$ (VIII)

worin eines von X und Y Wasserstoff ist und das andere eine Gruppe $Q(+O^-)L$ ist, worin Q Schwefel oder Selenium ist und L eine geeignete Gruppe bedeutet;

(d) Reaktion einer Verbindung der Formel (IX) mit einer Verbindung der Formel (X):

$Ar(CH_2)_nCH_2\text{-}Hal$ (IX)

$R^2C{\equiv}CR^3(CR^2{=}CR^3)C(O)NR^1$ (X)

worin Hal eine Halogenatom bedeutet, gefolgt von der Reduktion der Driefachbindung; oder

(e) Reaktion einer Verbindung der Formel (XI) mit einer Verbindung der Formel (XII):

$Ar(CH_2)_nCR^2{=}CR^3{—}M$ (XI)

O
‖
$Hal\text{-}(CR^2{=}CR^3)CNRR^1$ (XII)

worin Hal ein Halogenid bedeutet und M ein Metallatom oder eine Metallgruppe bedeutet.

11. Pestizide Zusammensetzung, enthaltend eine Verbindung

O
‖
$Ar(CH_2)_n(CR^2{=}CR^3)_2CNNR^1$

worin: Ar Phenyl, Naphthyl, Thienyl, Fluorenyl, Phenanthrenyl, Dibenzofuranyl oder eine polykernige Gruppe (A) bedeutet:

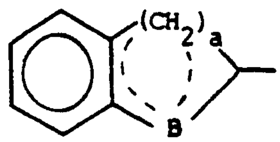

worin a 0, 1 oder 2 ist; B $(D)_b(CH_2)_c(E)_e$, worin jeder Rest von D und E Sauerstoff oder Schwefel ist, b und e unabhängig 0 oder 1 sind, aber nicht beide 1 sind, und c 0, 1, 2 oder 3 ist, wobei die Summe von a, b, c und e mindestens 2 ist, und der Ring, der B enthält, vollständig oder teilweise gesättigt ist; und G Wasserstoff oder einen Benzolring bedeutet, der an den Benzolring der Gruppe (A) kondensiert ist;

jeder Rest der Gruppe Ar kann durch eine oder mehrere von $C_{1-4}$-Alkyl, Halo-$(C_{1-4})$-alkyl, Halo, $C_{1-4}$-Alkoxy (ausgenommen 3,4-Methylendioxy)oder $C_{1-4}$-Halo-alkoxy substituiert sein; n 1 bis 8 ist, ausgenommen, daß n 1 bis 4 ist, wenn Ar Phenyl oder substituiertes Phenyl bedeutet;

jeder Rest von $R^2$ und $R^3$ in jedem Fall unabhängig Wasserstoff bedeutet, $C_{1-4}$-Alkyl oder Halo-$C_{1-4}$-alkyl; und R und $R^1$ jeweils aus Wasserstoff, Alkyl, Cycloalkyl, Alkenyl oder Alkoxy ausgewählt sind (jeder Rest kann durch Halo, Alkenyl, Alkyl, Cycloalkyl, Alkoxy, Alkinyl oder Cyano substituiert sein), mit der Ausnahme, daß die folgenden Verbindungen ausgeschlossen sind:

N-Isobutyl-6-(2-naphthyl)-hexa-2E,4E-dienamid
N-(2,2-Dimethylpropyl)-6-(2-naphthyl)-hexa-2E,4E-dienamid
N-Isobutyl-6-(2-fluorenyl)-hexa-hexa-2E,4E-dienamid
N-(2,2-Dimethyl-but-3-enyl)-6-(2-naphthyl)-hexa-hexa-2E,4E-dienamid
N-(2-Methylbutyl)-6-(2-naphthyl)-hexa-hexa-2E,4E-dienamid
N-Isobutyl-6-(2-phenanthrenyl)-hexa-2E,4E-dienamidhexa-2E,4E-dienamid
N-Isobutyl-6-(5-brom-2-naphthyl)-hexa-2E,4E-dienamid
N-Isobutyl-6-(6-brom-2-naphthyl)-hexa-2E,4E-dienamid
N-Isobutyl-6-(2-dibenzofuranyl)-hexa-2E,4E-dienamid
N-(1,2,2,-Trimethylpropyl)-6-(2-naphthyl)-hexa-2E,4E-dienamid
N-(1,2-Dimethylpropyl)-6-(2-naphthyl)-hexa-2E,4E-dienamid
N-(2,2-Dimethylpropyl-6-(2-phenanthrenyl)-hexa-2E,4E-dienamid
N-(1,2-Dimethylpropyl)-6-(2-phenanthrenyl)-hexa-2E,4E-dienamid
N-(2-Methylbutyl)-6-(2-phenanthrenyl)-hexa-2E,4E-dienamid
N-(2,2-Dimethylpropyl)-6-(5-brom-2-naphthyl)-hexa-2E,4E-dienamid
N-(2-Methylbutyl)-6-(5-brom-2-naphthyl)-hexa-2E,4E-dienamid
N-(2,2-Dimethylpropyl)-6-(2-dibenzofuranyl-hexa-2E,4E-dienamid
N-(2,2-Dimethylbut-3-enyl)-6-(2-dibenzofuranyl)-hexa-2E,4E-dienamid
N-(2,2-Dimethylbut-3-enyl)-6-(2-phenanthrenyl)-hexa-2E,4E-dienamid
N-(1,2-Dimethylpropyl)-6-(2-dibenzofuranyl)-hexa-2E,4E-dienamid
N-(2-Methylbutyl)-6-(2-dibenzofuranyl)-hexa-2E,4E-dienamid
N-(1,2-Dimethylpropyl-6-(5-brom-2-naphthyl)-hexa-2E,4E-dienamid
N-(2,2-Dimethylbut-3-enyl)-6-(5-brom-2-naphthyl)-hexa-2E,4E-dienamid
N-(2,2-Dimethylpropyl)-6-(9-brom-3-phenanthrenyl)-hexa-2E,4E-dienamid
N-(2,2-Dimethylpropyl)-6-(5,8-dibrom-2-naphthyl)-hexa-2E,4E-dienamid
N-(2,2-Dimethylpropyl)-6-(7-brom-2-naphthyl)-hexa-2E,4E-dienamid
N-(2,2-Dimethylpropyl)-6-(7-chlor-2-naphthyl)-hexa-2E,4E-dienamid
N-(2-Methylpropyl)-6-(7-chlor-2-naphthyl)-hexa-2E,4E-dienamid
N-(2,2-Dimethylpropyl)-6-(7-fluor-2-naphthyl)-hexa-2E,4E-dienamid
N-(2,2-Dimethylbut-3-enyl)-6-(9,10-dilydrophenanthrenyl)-hexa-2E,4E-dienamid
N-(1,2-Dimethylpropyl)-6-(7-fluor-2-naphthyl)-hexa-2E,4E-dienamid
N-Isobutyl-8-phenyl-octa-(2E,4E)-dienamid
N-Isobutyl-6-phenyl-hexa-(2E,4E)-dienamid.
N-Isobutyl-6-(2-thienyl)-hexa-(2E,4E)-dienamid
und einen oder mehrere Trägerstoffe.

12. Verfahren zur Bekämpfung von Schädlingen durch Applikation an eine Stelle einer Verbindung nach einem der Ansprüche 1 bis 9 oder einer Zusammensetzung nach Anspruch 11.

13. N-(1,2-Dimethylpropyl)-6-(indan-2-yl)-hexa-2,4-dienamid.

**Patentansprüche für den Vertragsstaat: AT**

1. Verfahren zur Herstellung einer Verbindung der Formel (I):

$$Ar(CH_2)_n(CR^2{=}CR^3)_2NRR^1 \qquad (I)$$

worin: Ar Phenyl, Naphthyl, Thienyl, Fluorenyl, Phenanthrenyl, Dibenzofuranyl oder eine polykernige Gruppe (A) bedeutet:

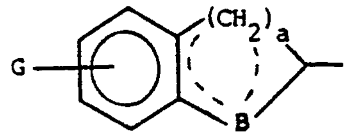

worin a 0, 1 oder 2 ist; B $(D)_b(CH_2)_c(E)_e$ ist, worin jeder Rest D und E Sauerstoff oder Schwefel ist, b und e unabhängig 0 oder 1 sind, aber nicht beide 1 sind, und c 0, 1, 2 oder 3 ist, wobei die Summe von a, b, c und e mindestens 2 ist, und der Ring, der B enthält, vollstandig oder teilweise gesättigt ist; und G Wasserstoff oder einen Benzolring beduetet, der an den Benzolring der Gruppe (A) kondensiert ist;

jede Gruppe Ar kann durch eine oder mehrere von $C_{1-4}$-Alkyl, Halo-($C_{1-4}$)-alkyl, Halo, $C_{1-4}$-Alkoxy (ausgenommen 3,4-Methylendioxy) oder $C_{1-4}$-Halo-alkoxy substituiert sein; n ist 1 bis 8, ausgenommen, daß n 1 bis 4 ist, wenn Ar Phenyl oder substituiertes Phenyl bedeutet;

jeder Rest von $R^2$ und $R^3$ in jedem Fall unabhängig Wasserstoff, $C_{1-4}$-Alkyl oder Halo-$C_{1-4}$-alkyl bedeutet; und R und $R^1$ jeweils aus Wasserstoff, $C_{1-6}$-Alkyl, $C_{3-6}$-Cycloalkyl, $C_{2-6}$-Alkenyl oder $C_{1-6}$-Alkoxy ausgewählt sind (jeder Rest kann durch Halo, $C_{2-6}$-Alkenyl, $C_{1-6}$-Alkyl, $C_{3-6}$Cycloalkyl, $C_{1-6}$-Alkoxy, $C_{2-6}$-Alkinyl oder Cyano substituiert sein), ausgenommen die folgenden Verbindungen:

N-Isobutyl-7-phenyl-hepta-(2E,4E)-dienamid
N-Isobutyl-6-phenyl-hexa-(2E,4E)-dienamid
N-Isobutyl-6-(4-methoxyphenyl)-hexa-(2E,4E)-dienamid
N-Isobutyl-6-(2-thienyl)-hexa-(2E,4E)-dienamid
N-Isobutyl-8-phenylocty-(2E,4E)-dienamid
N-Isobutyl-6-(1-naphthyl)-hexa-(2E,4E)-dienamid

durch:

(a) Reaktion einer Verbindung der Formel (II) mit einer Verbindung der Formel (III):

$$Ar(CH_2)_n(CR^2{=}CR^3)_2\ COZ^1 \qquad (II)$$

$$HNRR^1 \qquad (III)$$

worin $Z^1$ Hydroxyl, Halogen oder eine Phosphorimidatestergruppe (—$P(O^-)$(O Aryl) NH Aryl) ist und die anderen Variablen wie oben definiert sind;

(b) Reaktion einer Verbindung der Formel (IV) mit einer Verbindung der Formel (V) oder (VI):

$$Ar(CH_2)_n(CR^2{=}CR^3)_pC(O)H \qquad (IV)$$

$$(Z'')_3P{=}CH(CR^2{=}CHR^3)_qC(O)NRR^1 \qquad (V)$$

$$(Z'')_2\underset{\substack{|\\ O^-}}{P}{=}CH(CR^2{=}CHR^3)_qC(O)NRR^1 \qquad (VI)$$

worin Z'' Alkyl, Alkoxy (vorzugsweise Ethoxy) oder Aryl (vorzugsweise Phenyl) bedeutet, und p + q = 1. Die Stellungen der aldehyd- und phosphorhaltigen Gruppen, $(Z'')_3O$ und $(Z'')_2P(O)$, können ausgetauscht werden, um eine exakt analoge Reaktion zu ergeben;

(c) β-Elimination aus einer Verbindung der Formel (VII) oder (VIII):

$$Ar(CH_2)_n(CR^2{=}CR^3)\overset{\substack{X\\ |}}{C}R^2\overset{\substack{Y\\ |}}{C}R^3C(O)NRR^1 \qquad (VII)$$

$$Ar(CH_2)_n\overset{\substack{X\\ |}}{C}R^2\overset{\substack{Y\\ |}}{C}R^3(CR^2{=}CR^3)C(O)NRR^1 \qquad (VIII)$$

worin eines von X und Y Wasserstoff ist und das andere eine Gruppe $Q(+O^-)L$ ist, worin Q Schwefel oder Selenium ist und L eine geeignete Gruppe bedeutet;

(d) Reaktion einer Verbindung der Formel (IX) mit einer Verbindung der Formel (X):

$$Ar(CH_2)_nCH_2\text{-}Hal \qquad (IX)$$

$$R^2C{\equiv}CR^3(CR^2{=}CR^3)C(O)NR^1 \qquad (X)$$

worin Hal eine Halogenatom bedeutet, gefolgt von der Reduktion der Dreifachbindung; oder

(e) Reaktion einer Verbindung der Formel (XI) mit einer Verbindung der Formel (XII):

$$Ar(CH_2)_nCR^2{=}CR^3{-}M \qquad (XI)$$

$$Hal\text{-}(CR^2{=}CR^3)\overset{\overset{\displaystyle O}{\|}}{C}NRR^1 \qquad (XII)$$

worin Hal ein Halogenid bedeutet und M ein Metallatom oder eine Metallgruppe bedeutet.

2. Verfahren nach Anspruch 1, worin n eine ungerade Zahl ist.

3. Verfahren nach Anspruch 1 oder 2, worin die Konfiguration von beiden Doppelbindungen in der Diengruppe E ist.

4. Verfahren nach einem der Ansprüche 1 bis 3, worin Ar Phenyl, Furyl, Thienyl, Naphthyl, Benzofuranyl, Benzopyranyl, Chromanyl, Indanyl, Tetrahydronaphthyl oder eine der folgenden Gruppe bedeutet:

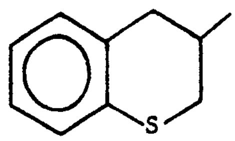

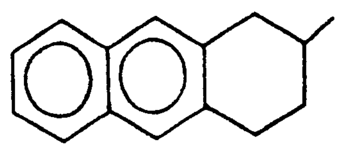

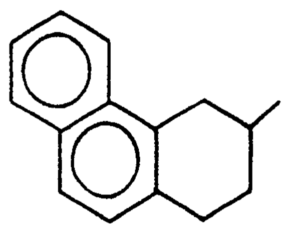

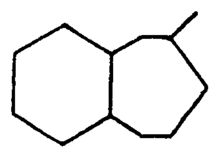

wobei jede davon wie in den Ansprüchen 1 bis 3 angegeben substituiert sein kann.

5. Verfahren nach Anspruch 4, worin Ar Phenyl oder in der 3-Stellung durch Halogen, Halogenalkyl oder Alkoxy substituiertes Phenyl bedeutet, oder Ar 3,4-Dihalogenphenyl ist.

6. Verfahren nach einem der Ansprüche 1 bis 5, worin R Wasserstoff bedeutet und $R^1$ Isobutyl, 1-Methylpropyl, 2,2-Dimethylpropyl, 1,2,2-Trimethylpropyl oder 1,2-Dimethylpropyl bedeutet.

7. Verfahren nach einem der Ansprüche 1 bis 6, worin $R^2$ und $R^3$ in jedem Fall Wasserstoff bedeuten.

8. Pestizide Zusammensetzung, enthaltend eine Verbindung, hergestellt nach einem der Ansprüche 1 bis 7 und einen oder mehrere Trägerstoff.

9. Verfahren zur Bekämpfung von Schädlingen durch Applikation an einem Ort, wobei eine Verbindung, hergestellt nach einem der Ansprüche 1 bis 7 oder eine Zusammensetzung nach Anspruch 1, aufgebracht wird.

10. N-(1,2-Dimethylpropyl)-6-(indan-2-yl)-hexa-2,4-dienamid.

**Patentansprüche für die Vertragsstaaten: SE LU**

1. Verbindung der Formel (I):

$$Ar(CH_2)_n(CR^2{=}CR^3)_2\overset{\overset{\displaystyle O}{\|}}{C}_{NRR^1} \qquad (I)$$

worin: Ar Phenyl, Naphthyl, Thienyl, Fluorenyl, Phenanthrenyl, Dibenzofuranyl oder eine polykernige Gruppe (A) bedeutet:

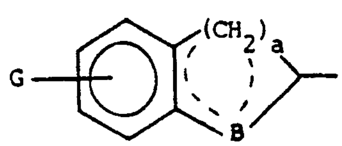

worin a 0, 1 oder 2 ist; B $(D)_b(CH_2)_c(E)_e$ ist, worin jeder Rest D und E Sauerstoff oder Schwefel ist, b und e unabhängig 0 oder 1 sind, aber nicht beide 1 sind, und c 0, 1, 2 oder 3 ist, wobei die Summe von a, b, c und e mindestens 2 ist, und der Ring, der B enthält, vollstandig oder teilweise gesättigt ist; und G Wasserstoff oder einen Benzolring bedeutet, der an den Benzolring der Gruppe (A) kondensiert ist;

jede Gruppe Ar kann durch eine oder mehrere von $C_{1-4}$-Alkyl, Halo-($C_{1-4}$)-alkyl, Halo, $C_{1-4}$-Alkoxy

(ausgenommen 3,4-Methylendioxy) oder $C_{1-4}$-Halo-alkoxy substituiert sein; n ist 1 bis 8, ausgenommen, daß n 1 bis 4 ist, wenn Ar Phenyl oder substituiertes Phenyl bedeutet;

jeder Rest von $R^2$ und $R^3$ in jedem Fall unabhängig Wasserstoff, $C_{1-4}$-Alkyl oder Halo-$C_{1-4}$-alkyl bedeutet; und R und $R^1$ jeweils aus Wasserstoff, $C_{1-6}$-Alkyl, $C_{3-6}$-Cycloalkyl, $C_{2-6}$-Alkenyl oder $C_{1-6}$-Alkoxy ausgewählt sind (jeder Rest kann durch Halo, $C_{2-6}$-Alkenyl, $C_{1-6}$-Alkyl, $C_{3-6}$-Cycloalkyl, $C_{1-6}$-Alkoxy, $C_{2-6}$-Alkinyl oder -Cyano substituiert sein), mit der Ausnahme, daß die folgenden Verbindungen ausgeschlossen sind:

N-Isobutyl-7-phenyl-hepta-(2E,4E)-dienamid
N-Isobutyl-6-phenyl-hexa-(2E,4E)-dienamid
N-Isobutyl-6-(4-methoxyphenyl)-hexa-(2E,4E)-dienamid
N-Isobutyl-6-(2-thenyl)-hexa-(2E,4E)-dienamid
N-Isobutyl-8-phenylocta-(2E,4E)-dienamid
N-Isobutyl-6-(1-naphthyl)-hexa-(2E,4E)-dienamid.

2. Verbindung nach Anspruch 1, worin n eine ungerade Zahl ist.

3. Verbindung nach Anspruch 1, worin die Konfiguration von beiden Doppelbindungen in der Diengruppe E ist.

4. Verbindung nach einem der Ansprüche 1 bis 3, worin Ar Phenyl, Furyl, Thienyl, Benzofuranyl, Benzopyranyl, Chromanyl, Indanyl, Tetrahydronaphthyl oder eine der folgenden Gruppen bedeutet:

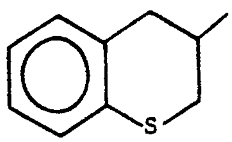
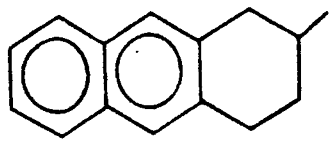
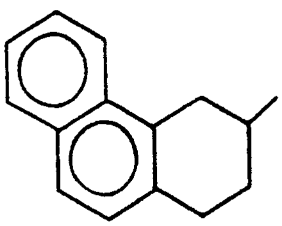
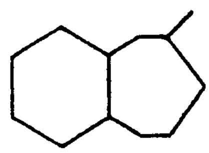

wobei jede wie in den Ansprüchen 1 bis 3 angegeben substituiert sein kann.

5. Verbindung nach Anspruch 4, worin Ar Phenyl oder in der 3-Stellung durch Halogen, Halogenalkyl oder Alkoxy substituiertes Phenyl bedeutet, oder Ar 3,4-Dihalogenphenyl bedeutet.

6. Verbindung nach einem der Ansprüche 1 bis 5, worin R Wasserstoff bedeutet und $R^1$ Isobutyl, 1-Methylpropyl, 2,2-Dimethylpropyl, 1,2,2-Trimethylpropyl oder 1,2-Dimethylpropyl bedeutet.

7. Verbindung nach einem der Ansprüche 1 bis 6, worin $R^2$ und $R^3$ in jedem Fall Wasserstoff bedeuten.

8. Verfahren zur Herstellung einer Verbindung nach einem der Ansprüche 1 bis 7 durch:

(a) Reaktion einer Verbindung der Formel (II) mit einer Verbindung der Formel (III):

$$Ar(CH_2)_n(CR^2{=}CR^3)_2\,COZ^1 \qquad (II)$$

$$HNRR^1 \qquad (III)$$

worin $Z^1$ Hydroxyl, Halogen oder eine Phosphorimidatestergruppe

$$(\text{—}\overset{O^-}{\overset{|}{P}}\ (O\ Aryl)\ NH\text{-}Aryl)$$

bedeutet und die anderen Variablen die in Anspruch 1 gegebene Bedeutung aufweisen;

(b) Reaktion einer Verbindung der Formel (IV) mit einer Verbindung der Formel (V) oder (VI):

$$Ar(CH_2)_n(CR^2{=}CR^3)_p\overset{O}{\overset{\|}{C}}H \qquad (IV)$$

$$(Z'')_3P{=}CH(CR^2{=}CHR^3)_qC(O)NRR^1 \qquad (V)$$

$$\underset{\substack{|\\ O^-}}{(Z'')_2P}=CH(CR^2=CR^3)_qC(O)NRR^1 \qquad (VI)$$

worin Z'' Alkyl, Alkoxy (vorzugsweise Ethoxy) oder Aryl (vorzugsweise Phenyl) bedeutet, und p + q = 1. Die Stellungen der aldehyd- und phosphorhaltigen Gruppen, $(Z'')_3O$ und $(Z'')_2P(O)$, können ausgetauscht werden, um eine exakt analoge Reaktion zu ergeben;

(c) β-Elimination aus einer Verbindung der Formel (VII) oder (VIII):

$$Ar(CH_2)_n(CR^2=CR^3)\overset{\substack{X\\ |}}{C}R^2\overset{\substack{Y\\ |}}{C}R^3C(O)NRR^1 \qquad (VII)$$

$$Ar(CH_2)_n\overset{\substack{X\\ |}}{C}R^2\overset{\substack{Y\\ |}}{C}R^3(CR^2=CR^3)C(O)NRR^1 \qquad (VIII)$$

worin eines von X und Y Wasserstoff ist und das andere eine Gruppe $Q(\rightarrow O^-)L$ ist, worin Q Schwefel oder Selenium ist und L eine geeignete Gruppe bedeutet;

(d) Reaktion einer Verbindung der Formel (IX) mit einer Verbindung der Formel (X):

$$Ar(CH_2)_nCH_2\text{-}Hal \qquad (IX)$$

$$R^2C\equiv CR^3(CR^2=CR^3)C(O)NR^1 \qquad (X)$$

worin Hal ein Halogenatom bedeutet, gefolgt von der Reduktion der Dreifachbindung; oder

(e) Reaktion einer Verbindung der Formel (XI) mit einer Verbindung der Formel (XII):

$$Ar(CH_2)_nCR^2=CR^3\text{—}M \qquad (XI)$$

$$Hal\text{-}(CR^2=CR^3)\overset{\substack{O\\ \|}}{C}NRR^1 \qquad (XII)$$

worin Hal ein Halogenid bedeutet und M ein Metallatom oder eine Metallgruppe bedeutet.

9. Pestizide Zusammensetzung, enthaltend eine Verbindung

$$Ar(CH_2)_n(CR^2=CR^3)_2\overset{\substack{O\\ \|}}{C}NRR^1$$

worin: Ar Phenyl, Naphthyl, Thienyl, Fluorenyl, Phenanthrenyl, Dibenzofuranyl oder eine polykernige Gruppe (A) bedeutet:

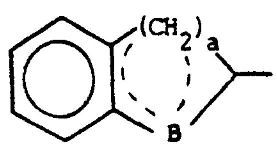

worin a 0, 1 oder 2 ist; B $(D)_b(CH_2)_c(E)_e$ ist, worin jeder Rest D und E Sauerstoff oder Schwefel ist, b und e unabhängig 0 oder 1 sind, aber nicht beide 1 sind, und c 0, 1, 2 oder 3 ist, wobei die Summe von a, b, c und e mindestens 2 ist, und der Ring, der B enthält, vollstandig oder teilweise gesättigt ist; und G Wasserstoff oder einen Benzolring bedeutet, der an den Benzolring der Gruppe (A) kondensiert ist;

jeder Gruppe Ar kann durch eine oder mehrere von $C_{1-4}$-Alkyl, Halo-$(C_{1-4})$-alkyl, Halo, $C_{1-4}$-Alkoxy (ausgenommen 3,4-Methylendioxy) oder $C_{1-4}$-Halo-alkoxy substituiert sein; n ist 1 bis 8, ausgenommen, daß n 1 bis 4 ist, wenn Ar Phenyl oder substituiertes Phenyl bedeutet;

jeder Rest von $R^2$ und $R^3$ ist in jedem Fall unabhängig Wasserstoff, $C_{1-4}$-Alkyl oder Halo-$C_{1-3}$-alkyl; und R und $R^1$ jeweils aus Wasserstoff, $C_{1-6}$-Alkyl, $C_{3-6}$-Cycloalkyl, $C_{2-6}$-Alkenyl oder $C_{1-4}$-Alkoxy ausgewählt sind (jeder Rest kann durch Halo, $C_{2-6}$-Alkenyl, $C_{1-6}$-Alkyl, $C_{3-6}$-Cycloalkyl, $C_{1-6}$-Alkoxy, $C_{2-6}$-Alkinyl oder Cyano substituiert sein), und einen oder mehrere Trägerstoffe.

10. Verfahren zur Bekämpfung von Schädlingen durch Applikation an einen Ort einer Verbindung nach einem der Asprüche 1 bis 8 oder einer Zusammensetzung nach Anspruch 9.

11. N-(1,2-Dimethylpropyl)-6-(indan-2-yl)-hexa-2,4-dienamid.

**Revendications pour les Etats contractants: BE CH DE FR GB IT LI NL**

1. Composé de formule (I):

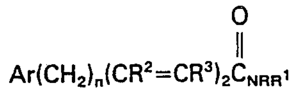

(I)

où Ar est un radical phényle, naphtyle, thiényle, fluorényle, phénanthrényle ou dibenzofurannyle ou un radical polynucléaire (A):

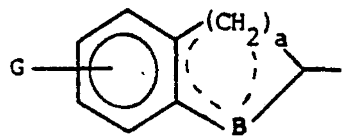

où a vaut 0, 1 ou 2; B représente $(D)_b(CH_2)_c(E)_e$ où chacun d'entre D et E représente l'oxygène ou le soufre, b et e valent indépendamment 0 ou 1, mais pas tous deux 1, et c vaut 0, 1, 2 ou 3, la somme de a, b, c et e étant d'au moins 2, et le cycle contenant B est complètement ou partiellement saturé; et G représente un atome d'hydrogène ou un cycle benzène condensé sur le cycle benzène du radical (A);

l'un quelconque des redicaux Ar peut être substitué par un ou plusieurs d'entre les radicaux $C_{1-4}$ alcoyle, halo-$(C_{1-4})$alcoyle, halo, $C_{1-4}$ alcoxy (à l'exception de 3,4-méthoylènedioxy) et halo-($C_{1-4}$ alcoxy); n vaut 1 à 8, sauf que n vaut 1 à 4 lorsque Ar représente un radical phényle ou phényle substitué;

chacun d'entre $R^2$ et $R^3$ représente dans chaque cas indépendamment un atome d'hydrogène ou radical $C_{1-4}$ alcoyle ou halo-$(C_{1-4})$alcoyle; et R et $R^1$ sont chacun choisis parmi les atomes d'hydrogène et radicaux $C_{1-6}$ alcoyle, $C_{3-6}$ cycloalcoyle, $C_{2-6}$ alcényle et $C_{1-6}$ alcoxy (dont l'un quelconque peut être substitué par halo, $C_{2-6}$ alcényle, $C_{1-6}$ alcoyle, $C_{3-6}$ cycloalcoyle, $C_{1-6}$ alcoxy, $C_{2-6}$ alcynyle ou cyano), à l'exception des composés suivants:

le N-isobutyl-6-(2-naphtyl)hexa-2E,4E-diénamide
le N-(2,2-diméthylpropyl)-6-(2-naphtyl)hexa-2E,4E-diénamide
le N-isobutyl-6-(2-fluorényl)hexa-2E,4E-diénamide
le N-(2,2-diméthyl-but-3-ényl)-6-(2-naphtyl)hexa-2E,4E-diénamide
le N-(2-méthylbutyl)-6-(2-naphtyl)hexa-2E,4E-diénamide
le N-isobutyl-6-(2-phénanthrényl)hexa-2E,4E-diénamide
le N-isobutyl-6-(5-bromo-2-naphtyl)hexa-2E,4E-diénamide
le N-isobutyl-6-(6-bromo-2-naphtyl)hexa-2E,4E-diénamide
le N-isobutyl-6-(2-dibenzofurannyl)hexa-2E,4E-diénamide
le N-(1,2,2,-triméthylpropyl)-6-(2-naphtyl)hexa-2E,4E-diénamide
le N-(1,2-diméthylpropyl)-6-(2-naphtyl)hexa-2E,4E-diénamide
le N-(2,2-diméthylpropyl-6-(2-phénanthrényl)hexa-2E,4E-diénamide
le N-(1,2-diméthylpropyl)-6-(2-phénanthrényl)hexa-2E,4E-diénamide
le N-(2-méthylbutyl)-6-(2-phénanthrényl)hexa-2E,4E-diénamide
le N-(2,2-diméthylpropyl)-6-(5-bromo-naphtyl)hexa-2E,4E-diénamide
le N-(2-méthylbutyl)-6-(5-bromo-2-naphtyl)hexa-2E,4E-diénamide
le N-(2,2-diméthylpropyl)-6-(2-dibenzofurannyl)hexa-2E,4E-diénamide
le N-(2,2-diméthylbut-3-ényl)-6-(2-dibenzofurannyl)hexa-2E,4E-diénamide
le N-(2,2-diméthylbut-3-ényl)-6-(2-phénanthrényl)hexa-2E,4E-diénamide
le N-(1,2-diméthylpropyl)-6-(2-dibenzofurannyl)hexa-2E,4E-diénamide
le N-(2-méthylbutyl)-6-(2-dibenzofurannyl)hexa-2E,4E-diénamide
le N-(1,2-diméthylpropyl-6-(5-brom-2-naphtyl)hexa-2E,4E-diénamide
le N-(2,2-diméthylbut-3-ényl)-6-(5-bromo-2-naphtyl)hexa-2E,4E-diénamide
le N-(2,2-diméthylpropyl)-6-(9-bromo-3-phénanthrényl)hexa-2E,4E-diénamide
le N-(2,2-diméthylpropyl)-6-(5,8-dibromo-2-naphtyl)hexa-2E,4E-diénamide
le N-(2,2-diméthylpropyl)-6-(7-bromo-2-naplhtyl)hexa-2E,4E-diénamide
le N-(2,2-diméthylpropyl)-6-(7-chloro-2-naphtyl)hexa-2E,4E-diénamide
le N-(2-méthylpropyl)-6-(7-chloro-2-naphtyl)hexa-2E,4E-diénamide
le N-(2,2-diméthylpropyl)-6-(7-fluoro-2-naphtyl)hexa-2E,4E-diénamide
le N-(2,2-dimeethylbut-3-ényl)-6-(9,10-dihydrophénanthrényl)hexa-2E,4E-diénamide
le N-(1,2-diméthylpropyl)-6-(7-fluoro-2-naphtyl)hexa-2E,4E-diénamide
le N-(3,3-diméthylpropyl)-6-(6-chloro-2-naphtyl)hexa-2E,4E-diénamide
le N-isobutyl-7-phénylhepta-(2E,4E)-diénamide
le N-isobutyl-6-phénylhexa-(2E,4E)-diénamide
le N-isobutyl-6-(4-méthoxyphényl)hexa-(2E,4E)-diénamide
le N-isobutyl-6-(2-thiényl)hexa-(2E,4E)-diénamide
le N-isobutyl-8-phénylocta-(2E,4E)-diénamide
le N-isobutyl-6-(1-naphtyl)hexa-(2E,4E)-diénamide.

2. Composé de formule (IA):

$$Ar\text{-}(CH_2)_n(CR^2{=}CR^3)_2\ C(O)NRR^1 \qquad (IA)$$

où Ar, n, R, $R^1$, $R^2$ et $R^3$ sont tels que définis ci-dessus, sauf que (i) lorsque n représente 1 et que R représente H et Ar est un radical 2-fluorényle, 2-phénanthrényle, 2-dibenzofurannyle, 9,10-dihydro-2-phénanthrényle, 5-, 6- ou 7-halo-2-naphtyle, 5,8-dibromo-2-naphtyle ou 3-(9-bromo)phénanthrényle, alors $R^1$ n'est pas un radical isobutyle, 2,2-diméthylpropyle, 2,2-diméthyl-3-butényle, 2-méthylbutyle, 1,2,2-triméthylpropyle ou 1,2-diméthylpropyle, et (ii) lorsque n est 1 et que R représente H et Ar est un radical 2-naphtyle, alors $R^1$ n'est pas un radical 2,2-diméthylpropyle, 2,2-diméthyl-3-butényle, 2-méthylbutyle ou 1,2,2-triméthylpropyle.

3. Composé de formule (IB):

$$Ar{-}(CH_2)_n(CR^2{=}CR^3)_2C(O)NRR^1 \qquad (IB)$$

où Ar, n, R, $R^1$, $R^2$ et $R^3$ sont tels que définis ci-dessus, sauf que lorsque n représente 1 et que R représente H et Ar est soit un système polycyclique totalement aromatique uni à la position 2 soit un radical 3-phénanthrényle, alors $R^1$ n'est pas un radical isobutyle, 2,2-diméthylpropyle, 2,2-diméthyl-3-butényle, 2-méthylbutyle, 1,2,2-triméthylpropyle ou 1,2-diméthylpropyle, avec la restriction que le N-isobutyl-8-(2-naphtyl)octa-2E,4E-diénamide et le N-(1,2-diméthylpropyl)-8-(2-naphtyl)octa-2E,4E-diénamide ne sont pas exclus.

4. Composé suivant l'une quelconque des revendications 1 à 3, dans lequel n est impair.

5. Composé suivant l'une quelconque des revendications 1 à 4, dans lequel la configuration des deux doubles liaisons dans le radical diénique est E.

6. Composé suivant l'une quelconque des revendications 1 à 5, dans lequel Ar est un radical phényle, furyle, thiényle, benzofurannyle, benzopyrannyle, chromanyle, indanyle, tétrahydronaphtyle ou l'un quelconque des radicaux suivants:

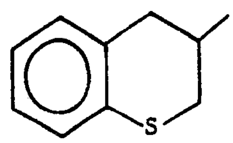
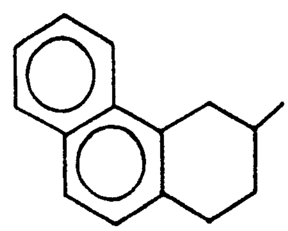
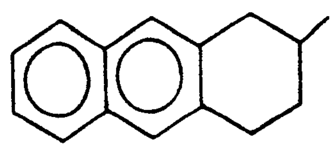
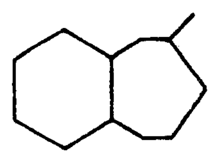

dont chacun peut être substitué comme dans les revendications 1 à 5.

7. Composé suivant la revendication 6, dans lequel Ar est un radical phényle ou phényle substitué à la position 3 par halo, haloalcoyle ou alcoxy ou bien Ar est un radical 3,4-dihalophényle.

8. Composé suivant l'une quelconque des revendications 1 à 5, dans lequel R est un atome d'hydrogène et $R^1$ est un radical isobutyle, 1-méthylpropyle, 2,2-diméthylpropyle, 1,2,2-triméthylpropyle ou 1,2-diméthylpropyle.

9. Composé suivant l'une quelconque des revendications 1 à 6, dans lequel $R^2$ et $R^3$ sont dans chaque cas une atome d'hydrogène.

10. Procédé de préparation d'un composé suivant l'une quelconque des revendications 1 à 9, par

(a) réaction d'un composé de formule (II) avec un composé de formule (III):

$$Ar(CH_2)_n(CR^2{=}CR^3)_2\ COZ' \qquad (II)$$

$$HNRR^1 \qquad (III)$$

où Z' est un radical hydroxyle, halo ou ester phosphoroimidate

$O^-$
|
(—P(O Aryl)NH Aryl)

et les autres variables sont telles que définies dans la revendication 1;

(b) réaction d'un composé de formule (IV) acec un composé de fomule (V) ou (VI):

$$Ar(CH_2)_n(CR^2=CR^3)_p\overset{\overset{\displaystyle O}{\|}}{C}H \qquad (IV)$$

$$(Z'')_3P=CH(CR^2=CHR^3)_qC(O)NRR^1 \qquad (V)$$

$$(Z'')_2\underset{\underset{\displaystyle O^-}{|}}{P}=CH(CR^2=CR^3)_qC(O)NRR^1 \qquad (VI)$$

où Z'' représente un radical alcoyle, alcoxy (de préférence éthoxy) ou aryle (de préférence phényle) et p + q = 1, les positions des radicaux aldéhyde et des radicaux contenant du phosphore $(Z'')_3P$ et $(Z'')_2P(O)$ pouvant être échangées pour donner une réaction exactement analogue;

(c) par β-élimination d'un composé de formule (VII) ou (VIII):

$$Ar(CH_2)_n(CR^2=CR^3)\overset{\overset{\displaystyle X}{|}}{C}R^2\overset{\overset{\displaystyle Y}{|}}{C}R^3C(O)NRR^1 \qquad (VII)$$

$$Ar(CH_2)_n\overset{\overset{\displaystyle X}{|}}{C}R^2\overset{\overset{\displaystyle Y}{|}}{C}R^3(CR^2=CR^3)C(O)NRR^1 \qquad (VIII)$$

où l'un d'entre X et Y représénte un atome d'hydrogène et l'autre représente un radical $Q(\rightarrow O^-)L$ où Q représente le soufre ou le sélénium et L représente un radical approprié;

(d) réaction d'une composé de formule (IX) avec un composé de formule (X):

$$Ar(CH_2)_nCH_2\text{-}Hal \qquad (IX)$$

$$R^2C\equiv CR^3(CR^2=CR^3)C(O)NR^1 \qquad (X)$$

où Hal est un atome d'halogène, suivie de la réduction de la triple liaison; ou

(e) réaction d'un composé de formule (XI) avec un composé de formule (XII):

$$Ar(CH_2)_nCR^2=CR^3\text{—}M \qquad (XI)$$

$$Hal\text{-}(CR^2=CR^3)\overset{\overset{\displaystyle O}{\|}}{C}NRR^1 \qquad (XII)$$

où Hal représente halogénure, et M est un atome métallique ou groupe métallique.

11. Composition pesticide comprenant un composé

$$Ar(CH_2)_n(CR^2=CR^3)_2C(O)NRR^1 \qquad (I)$$

où Ar est un radical phényle, naphtyle, thiényle, fuorényle, phénanthrényle ou dibenzofurannyle ou un radical polynucléaire (A):

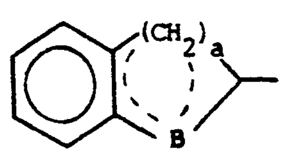

où a vaut 0, 1 ou 2;

B représente $(D)_b(CH_2)_c(E)_e$ où chacun d'entre D et E représente l'oxygène ou le soufre, b et e valent indépendamment 0 ou 1, mais pas tous deux 1, et c vaut 0, 1, 2 ou 3, la somme de a, b, c et e étant d'au moins 2, et le cycle contenant B est complètement ou partiellement saturé; et

G représente un atome d'hydrogène ou un cycle benzéne condensé sur le cycle benzène du radical (A);

l'un quelconque des redicaux Ar peut être substitué par un ou plusieurs d'entre les radicaux $C_{1-4}$ alcoyle, halo-$(C_{1-4})$alcoyle, halo, $C_{1-4}$ alcoxy (à l'exception de 3,4-méthoylènedioxy) et halo-($C_{1-4}$ alcoxy);

n vaut 1 à 8, sauf que n vaut 1 à 4 lorsque Ar représente un radical phényle ou phényle substitué;

chacun d'entre $R^2$ et $R^3$ représente dans chaque cas indépendamment un atome d'hydrogène ou radical $C_{1-4}$ alcoyle ou halo-($C_{1-4}$)alcoyle; et

R et $R^1$ sont chacun choisis parmi les atomes d'hydrogène et radicaux $C_{1-6}$ alcoyle, $C_{3-6}$ cycloalcoyle, $C_{2-6}$ alcényle et $C_{1-6}$ alcoxy (dont l'un quelconque peut être substitué par halo, $C_{2-6}$ alcényle, $C_{1-6}$ alcoyle, $C_{3-6}$ cycloalcoyle, $C_{1-6}$ alcoxy, $C_{2-6}$ alcynyle ou cyano),

à l'exception des composés suivants:

le N-isobutyl-6-(2-naphtyl)hexa-2E,4E-diénamide
le N-(2,2-diméthylpropyl)-6-(2-naphtyl)hexa-2E,4E-diénamide
le N-isobutyl-6-(2-fluorényl)hexa-2E,4E-diénamide
le N-(2,2-diméthyl-but-3-ényl)-6-(2-naphtyl)hexa-2E,4E-diénamide
le N-(2-méthylbutyl)-6-(2-naphtyl)hexa-2E,4E-diénamide
le N-isobutyl-6-(2-phénanthrényl)hexa-2E,4E-diénamide
le N-isobutyl-6-(5-bromo-2-naphtyl)hexa-2E,4E-diénamide
le N-isobutyl-6-(6-bromo-2-naphtyl)hexa-2E,4E-diénamide
le N-isobutyl-6-(2-dibenzofurannyl)hexa-2E,4E-diénamide
le N-(1,2,2,-triméthylpropyl)-6-(2-naphtyl)hexa-2E,4E-diénamide
le N-(1,2-diméthylpropyl)-6-(2-naphtyl)hexa-2E,4E-diénamide
le N-(2,2-diméthylpropyl-6-(2-phénanthrényl)hexa-2E,4E-diénamide
le N-(,2-diméthylpropyl)-6-(2-phénanthrényl)hexa-2E,4E-diénamide
le N-(2-méthylbutyl)-6-(2-phénanthrényl)hexa-2E,4E-diénamide
le N-(2,2-diméthylpropyl)-6-(5-bromo-2-naphtyl)hexa-2E,4E-diénamide
le N-(2-méthylbutyl)-6-(5-bromo-2-naphtyl)hexa-2E,4E-diénamide
le N-(2,2-diméthylpropyl)-6-(2-dibenzofurannyl)hexa-2E,4E-diénamide
le N-(2,2-diméthylbut-3-ényl)-6-(2-dibenzofurannyl)hexa-2E,4E-diénamide
le N-(2,2-diméthylbut-3-ényl)-6-(2-phénanthrényl)hexa-2E,4E-diénamide
le N-(1,2-diméthylpropyl)-6-(2-dibenzofurannyl)hexa-2E,4E-diénamide
le N-(2-méthylbutyl)-6-(2-dibenzofurannyl)hexa-2E,4E-diénamide
le N-(1,2-diméthylpropyl-6-(5-brom-2-naphtyl)hexa-2E,4E-diénamide
le N-(2,2-diméthylbut-3-ényl)-6-(5-bromo-2-naphtyl)hexa-2E,4E-diénamide
le N-(2,2-diméthylpropyl)-6-(9-brom-3-phénanthrényl)hexa-2E,4E-diénamide
le N-(2,2-diméthylpropyl)-6-(5,8-dibromo-2-naphtyl)hexa-2E,4E-diénamide
le N-(2,2-diméthylpropyl)-6-(7-bromo-2-naphtyl)hexa-2E,4E-diénamide
le N-(2,2-diméthylpropyl)-6-(7-chloro-2-naphtyl)hexa-2E,4E-diénamide
le N-(2-méthylpropyl)-6-(7-chloro-2-naphthyl)hexa-2E,4E-diénamide
le N-(2,2-diméthylpropyl)-6-(7-fluoro-2-naphthyl)hexa-2E,4E-diénamide
le N-(2,2-diméthylbut-3-ényl)-6-(9,10-dihydrophénanthrényl)hexa-2E,4E-diénamide
le N-(1,2-diméthylpropyl)-6-(7-fluoro-2-naphtyl)hexa-2E,4E-diénamide
le N-isobutyl-8-phénylocta-(2E,4E)-diénamide
le N-isobutyl-6-phénylhexa-(2E,4E)-diénamide
le N-isobutyl-6-(2-thiényl)hexa-(2E,4E)-diénamide

et un ou plusieurs excipients.

12. Procédé pour combattre des organismes nuisibles par application en un lieu d'un composé suivant l'une quelconque des revendications 1 à 9 ou d'un composition suivant la revendication 11.

13. Le N-(1,2-diméthylpropyl)-6-(indan-2-yl)hexa-2,4-diénamide.

**Revendications pour l'Etat contractant: AT**

1. Procédé de préparation d'un composé de formule (I):

$$Ar(CH_2)_n(CR^2{=}CR^3)_2C(O)NRR^1 \quad (I)$$

où Ar est un radical phényle, naphtyle, thiényle, fluorényle, phénanthrényle ou dibenzofurannyle ou un radical polynucléaire (A):

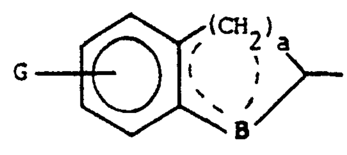

où a vaut 0, 1 ou 2; B représente $(D)_b(CH_2)_c(E)_e$ où chacun d'entre D et E représente l'oxygène ou le soufre, b et e valent indépendamment 0 ou 1, mais pas tous deux 1, et c vaut 0, 1, 2 ou 3, la somme de a, b, c et e étant d'au moins 2, et le cycle contenant B est complètement ou partiellement saturé; et G représente un atome d'hydrogène ou un cycle benzène condensé sur la cycle benzène du radical (A);

l'un quelconque des redicaux Ar peut être substitué par un ou plusieurs d'entre les radicaux $C_{1-4}$

alcoyle, halo-($C_{1-4}$)alcoyle, halo, $C_{1-4}$ alcoxy (à l'exception de 3,4-méthoylènedioxy) et halo-($C_{1-4}$ alcoxy); n vaut 1 à 8, sauf que n vaut 1 à 4 lorsque Ar représente un radical phényle ou phényle substitué;

chacun d'entre $R^2$ et $R^3$ représente dans chaque cas indépendamment un atome d'hydrogène ou radical $C_{1-4}$ alcoyle ou halo-($C_{1-4}$)alcoyle; et R et $R^1$ sont chacun choisis parmi les atomes d'hydrogène et radicaux $C_{1-6}$ alcoyle, $C_{3-6}$ cycloalcoyle, $C_{2-6}$ alcényle et $C_{1-6}$ alcoxy (dont l'un quelconque peut être substitué par halo, $C_{2-6}$ alcényle, $C_{1-6}$ alcoyle, $C_{3-6}$ cycloalcoyle, $C_{1-6}$ alcoxy, $C_{2-6}$ alcynyle ou cyano), a l'exception des composés suivants:

le N-isobutyl-7-phénylhepta-(2E,4E)-diénamide
le N-isobutyl-6-phénylhexa-(2E,4E)-diénamide
le N-isobutyl-6-(4-méthoxyphényl)hexa-(2E,4E)-diénamide
le N-isobutyl-6-(2-thiényl)hexa-(2E,4E)-diénamide
le N-isobutyl-8-phénylocta-(2E,4E)-diénamide
le N-isobutyl-6-(1-naphthyl)hexa-(2E,4E)-diénamide par

(a) réaction d'un composé de formule (II) avec un composé de formule (III):

$$Ar(CH_2)_n(CR^2{=}CR^3)_2\ COZ' \qquad (II)$$

$$HNRR^1 \qquad (III)$$

où Z' est un radical hydroxyle, halo ou ester phosphoroimidate

$$(\text{—}\overset{O^-}{\overset{|}{P}}(O\ Aryl)NH\ Aryl)$$

et les autres variables sont telles que définies ci-dessus;

(b) réaction d'un composé de formule (IV) avec un composé de formule (V) ou (VI):

$$Ar(CH_2)_n(CR^2{=}CR^3)_p\overset{O}{\overset{\|}{C}}H \qquad (IV)$$

$$(Z'')_3P{=}CH(CR^2{=}CHR^3)_qC(O)NRR^1 \qquad (V)$$

$$(Z'')_2\underset{O^-}{\underset{|}{P}}{=}CH(CR^2{=}CR^3)_qC(O)NRR^1 \qquad (VI)$$

où Z'' représente un radical alcoyle, alcoxy (de préférence éthoxy) ou aryle (de préférence phényle) et p + q = 1, les positions des radicaux aldéhyde et des radicaux contenant du phosphore $(Z'')_3P$ et $(Z'')_2P(O)$ pouvant être échangées pour donner une réaction exactement analogue;

(c) par β-élimination d'un composé de formule (VII) ou (VIII):

$$Ar(CH_2)_n(CR^2{=}CR^3)\overset{X}{\overset{|}{C}}R^2\overset{Y}{\overset{|}{C}}R^3C(O)NRR^1 \qquad (VII)$$

$$Ar(CH_2)_n\overset{X}{\overset{|}{C}}R^2\overset{Y}{\overset{|}{C}}R^3(CR^2{=}CR^3)C(O)NRR^1 \qquad (VIII)$$

où l'un d'entre X et Y représénte un atome d'hydrogène et l'autre représente un radical Q(→O$^-$)L où Q représente le soufre ou le sélénium et L représente un radical approprié;

(d) réaction d'une composé de formule (IX) avec un composé de formule (X):

$$Ar(CH_2)_nCH_2\text{-}Hal \qquad (IX)$$

$$R^2C{\equiv}CR^3(CR^2{=}CR^3)C(O)NR^1 \qquad (X)$$

où Hal est un atome d'halogène, suivie de la réduction de la triple liaison; ou

(e) réaction d'un composé de formule (XI) avec un composé de formule (XII):

$$Ar(CH_2)_nCR^2{=}CR^3\text{—}M \qquad (XI)$$

$$\text{Hal-}(CR^2{=}CR^3)\overset{\overset{\displaystyle O}{\|}}{C}NRR^1 \qquad (XII)$$

où Hal représente halogénure, et M est un atome métallique ou groupe métallique.

2. Procédé suivant la revendication 1, dans lequel n est impair.

3. Procédé suivant la revendication 1 ou 2, dans lequel la configuration des deux doubles liaisons dans le radical diénique est *E*.

4. Procédé suivant l'une quelconque des revendications 1 à 3, dans lequel Ar est un radical phényle, furyle, thiényle, benzofurannyle, benzopyrannyle, chromanyle, indanyle, tétrahydronaphtyle, naphtyle ou l'un quelconque des radicaux suivants:

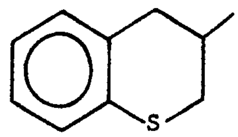

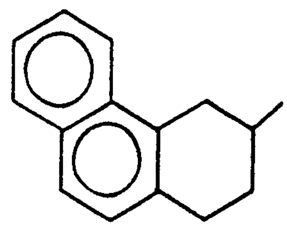

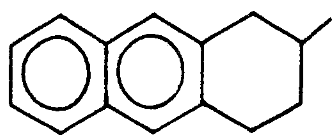

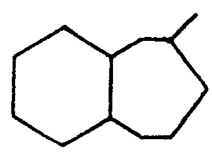

dont chacun peut être substitué comme dans les revendications 1 à 3.

5. Procédé suivant la revendication 4, dans lequel Ar est un radical phényle ou phényle substitué à la position 3 par halo, haloalcoyle ou alcoxy ou bien Ar est un radical 3,4-dihalophényle.

6. Composé suivant l'une quelconque des revendications 1 à 5, dans lequel R est un atome d'hydrogène et $R^1$ est un radical isobutyle, 1-méthylpropyle, 2,2-diméthylpropyle, 1,2,2-triméthylpropyle ou 1,2-diméthylpropyle.

7. Procédé suivant l'une quelconque des revendications 1 à 6, dans lequel $R^2$ et $R^3$ sont dans chaque cas une atome d'hydrogène.

8. Composition pesticide comprenant un composé préparé suivant l'une quelconque des revendications 1 à 7 et un ou plusieurs excipients.

9. Procédé pour combattre des organismes nuisibles par application en un lieu d'un composé suivant l'une quelconque des revendications 1 à 7 ou d'une composition suivant la revendication 8.

10. Le N-(1,2-diméthylpropyl)-6-(indan-2-yl)-hexa-2,4-diénamide.

**Revendications pour les Etats contractants: LU SE**

1. Composé de formule (I):

$$Ar(CH_2)_n(CR^2{=}CR^3)_2C(O)NRR^1 \qquad (I)$$

où Ar est un radical phényle, naphtyle, thiényle, fluorényle, phénanthrényle ou dibenzofurannyle ou un radical polynucléaire (A):

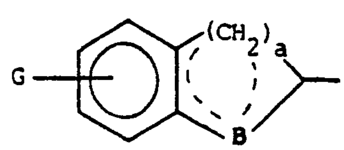

où a vaut 0, 1 ou 2; B représente $(D)_b(CH_2)_c(E)_e$ où chacun d'entre D et E représente l'oxygène ou le soufre, b et e valent indépendamment 0 ou 1, mais pas tous deux 1, et c vaut 0, 1, 2 ou 3, la somme de a, b, c et e étant d'au moins 2, et le cycle contenant B est complètement ou partiellement saturé; et G représente un atome d'hydrogène ou un cycle benzène condensé sur la cycle benzène du radical (A);

l'un quelconque des redicaux Ar peut être substitué par un ou plusieurs d'entre les radicaux $C_{1-4}$ alcoyle, halo-($C_{1-4}$)alcoyle, halo, $C_{1-4}$ alcoxy (à l'exception de 3,4-méthoylènedioxy) et halo-($C_{1-4}$ alcoxy); n

vaut 1 à 8, sauf que n vaut 1 à 4 lorsque Ar représente un radical phényle ou phényle substitué;

chacun d'entre $R^2$ et $R^3$ représente dans chaque cas indépendamment un atome d'hydrogène ou radical $C_{1-4}$ alcoyle ou halo-($C_{1-4}$)alcoyle; et R et $R^1$ sont chacun choisis parmi les atomes d'hydrogène et radicaux $C_{1-6}$ alcoyle, $C_{3-6}$ cycloalcoyle, $C_{2-6}$ alcényle et $C_{1-6}$ alcoxy (dont l'un quelconque peut être substitué par halo, $C_{2-6}$ alcényle, $C_{1-6}$ alcoyle, $C_{3-6}$ cycloalcoyle, $C_{1-6}$ alcoxy, $C_{2-6}$ alcynyle ou cyano), à l'exception des composés suivants:

le N-isobutyl-7-phénylhepta-(2E,4E)-diénamide

le N-isobutyl-6-phénylhexa-(2E,4E)-diénamide

le N-isobutyl-6-(4-méthoxyphényl)hexa-(2E,4E)-diénamide

le N-isobutyl-6-(2-thiényl)hexa-(2E,4E)-diénamide

le N-isobutyl-8-phénylocta-(2E,4E)-diénamide

le N-isobutyl-6-(1-naphthyl)hexa-(2E,4E)-diénamide

2. Composé suivant la revendication 1, dans lequel n est impair.

3. Composé suivant la revendication 1, dans lequel la configuration des deux doubles liaisons dans le radical diénique est *E*.

4. Composé suivant l'une quelconque des revendications 1 à 3, dans lequel Ar est un radical phényle, furyle, thiényle, benzofurannyle, benzopyrannyle, chromanyle, indanyle, tétrahydronaphtyle, naphtyle ou l'un quelconque des radicaux suivants:

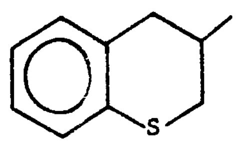

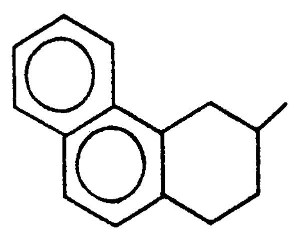

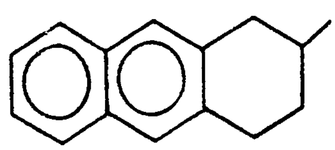

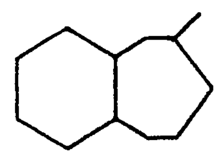

5. Composé suivant la revendication 4, dans lequel Ar est un radical phényle ou phényle substitué à la position 3 par halo, haloalcoyle ou alcoxy ou bien Ar est un radical 3,4-dihalophényle.

6. Composé suivant l'une quelconque des revendications 1 à 5, dans lequel R est un atome d'hydrogène et $R^1$ est un radical isobutyle, 1-méthylpropyle, 2,2-diméthylpropyle, 1,2,2-triméthylpropyle ou 1,2-diméthylpropyle.

7. Composé suivant l'une quelconque des revendications 1 à 6, dans lequel $R^2$ et $R^3$ sont dans chaque cas un atome d'hydrogène.

8. Procédé de préparation d'un composé suivant l'une quelconque des revendications 1 à 7, par

(a) réaction d'un composé de formule (II) avec un composé de formule (III):

$$Ar(CH_2)_n(CR^2{=}CR^3)_2\,COZ' \qquad (II)$$

$$HNRR^1 \qquad (III)$$

où $Z^1$ est un radical hydroxyle, halo ou ester phosphoroimidate

$$(-\overset{O^-}{\overset{|}{P}}(O\ Aryl)NH\ Aryl)$$

et les autres variables sont telles que définies dans la revendication 1;

(b) réaction d'un composé de formule (IV) avec un composé de formule (V) ou (VI):

$$Ar(CH_2)_n(CR^2{=}CR^3)_p\overset{\overset{O}{\|}}{C}H \qquad (IV)$$

$$(Z'')_3P{=}CH(CR^2{=}CHR^3)_qC(O)NRR^1 \qquad (V)$$

$$\begin{array}{l}(Z'')_2P{=}CH(CR^2{=}CR^3)_qC(O)NRR^1 \\ \;\;\;\;\;\;| \\ \;\;\;\;\;O^-\end{array} \qquad (VI)$$

où Z'' représente un radical alcoyle, alcoxy (de préférence éthoxy) ou aryle (de préférence phényle) et p + q = 1, les positions des radicaux aldéhyde et des radicaux contenant du phosphore $(Z'')_3P$ et $(Z'')_2P(O)$ pouvant être échangées pour donner une réaction exactement analogue;

(c) par β-élimination d'un composé de formule (VII) ou (VIII):

$$\begin{array}{c}X\;\;Y \\ |\;\;\;| \\ Ar(CH_2)_n(CR^2{=}CR^3)CR^2CR^3C(O)NRR^1\end{array} \qquad (VII)$$

$$\begin{array}{c}X\;\;Y \\ |\;\;\;| \\ Ar(CH_2)_nCR^2CR^3(CR^2{=}CR^3)C(O)NRR^1\end{array} \qquad (VIII)$$

où l'un d'entre X et Y représénte un atome d'hydrogène et l'autre représente un radical $Q(\rightarrow O^-)L$ où Q représente le soufre ou le sélénium et L représente un radical approprié;

(d) réaction d'une composé de formule (IX) avec un composé de formule (X):

$$Ar(CH_2)_nCH_2\text{-}Hal \qquad (IX)$$

$$R^2C{\equiv}CR^3(CR^2{=}CR^3)C(O)NR^1 \qquad (X)$$

où Hal est un atome d'halogène, suivie de la réduction de la triple liaison; ou

(e) réaction d'un composé de formule (XI) avec un composé de formule (XII):

$$Ar(CH_2)_nCR^2{=}CR^3{-}M \qquad (XI)$$

$$\begin{array}{c}O \\ \| \\ Hal\text{-}(CR^2{=}CR^3)CNRR^1\end{array} \qquad (XII)$$

où Hal représente halogénure, et M est un atome métallique que ou groupe métallique.

9. Composition pesticide comprenant un composé

$$Ar(CH_2)_n(CR^2{=}CR^3)_2C(O)NRR^1 \qquad (I)$$

où Ar est un radical phényle, naphtyle, thiényle, fluorényle, phénanthrényle ou dibenzofurannyle ou un radical polynucléaire (A):

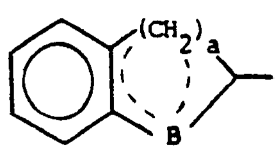

où a vaut 0, 1 ou 2;

B représente $(D)_b(CH_2)_c(E)_e$ où chacun d'entre D et E représente l'oxygène ou le soufre, b et e valent indépendamment 0 ou 1, mais pas tous deux 1, et c vaut 0, 1, 2 ou 3, la somme de a, b, c et e étant d'au moins 2, et le cycle contenant B est complètement ou partiellement saturé; et

G représente un atome d'hydrogène ou un cycle benzéne condensé sur la cycle benzéne du radical (A);

l'un quelconque des redicaux Ar peut être substitué par un ou plusieurs d'entre les radicaux $C_{1-4}$ alcoyle, halo-$(C_{1-4})$alcoyle, halo, $C_{1-4}$ alcoxy (à l'exception de 3,4-méthoylènedioxy) et halo-($C_{1-4}$ alcoxy);

n vaut 1 à 8, sauf que n vaut 1 à 4 lorsque Ar représente un radical phényle ou phényle substitué;

chacun d'entre $R^2$ et $R^3$ représente dans chaque cas indépendamment un atome d'hydrogène ou radical $C_{1-4}$ alcoyle ou halo-$(C_{1-4})$alcoyle; et

R et $R^1$ sont chacun choisis parmi les atomes d'hydrogène et radicaux $C_{1-6}$ alcoyle, $C_{3-6}$ cycloalcoyle, $C_{2-6}$ alcényle et $C_{1-6}$ alcoxy (dont l'un quelconque peut être substitué par halo, $C_{2-6}$ alcényle, $C_{1-6}$ alcoyle, $C_{3-6}$ cycloalcoyle, $C_{1-6}$ alcoxy, $C_{2-6}$ alcynyle ou cyano), et un ou plusieurs excipients.

10. Procédé pour combattre des organismes nuisibles par application en un lieu d'un composé suivant l'une quelconque des revendications 1 à 8 ou d'une composition suivant la revendication 9.

11. Le N-(1,2-diméthylpropyl)-6-(indan-2-yl)hexa-2,4-diénamide.